(19) Europäisches Patentamt
European Patent Office
Office européen des brevets

(11) Numéro de publication : **0 379 413 B1**

(12) # FASCICULE DE BREVET EUROPEEN

(45) Date de publication du fascicule du brevet : **22.12.93 Bulletin 93/51**

(21) Numéro de dépôt : **90400105.4**

(22) Date de dépôt : **15.01.90**

(51) Int. Cl.$^5$ : **C07D 471/04,** A61K 31/435, // (C07D471/04, 221:00, 221:00)

(54) **Dérivés de benzonaphtyridine-1,8, leur préparation et les compositions qui les contiennent.**

(30) Priorité : **16.01.89 FR 8900430**
**28.07.89 FR 8910219**

(43) Date de publication de la demande : **25.07.90 Bulletin 90/30**

(45) Mention de la délivrance du brevet : **22.12.93 Bulletin 93/51**

(84) Etats contractants désignés : **AT BE CH DE DK ES FR GB GR IT LI LU NL SE**

(56) Documents cités :
**EP-A- 0 132 845**
**EP-A- 0 379 412**
**CHEMICAL ABSTRACTS, vol. 61, no. 9, 26 octobre 1964, résumé no. 10666g, Columbus, Ohio, US; A.K. MALLAMS: "New synthesis of dibenzo[b,g][1,8]naphthyridines"**
**CHEMISCHE BERICHTE, vol. 106, no. 11, 1973, pages 3533-3539; L. JOCHEN et al.: "Heterocyclische beta-Enamino-ester. X. Zur Reaktivität von 2-Amino-3-chinolincarbonsäure-äthylester gegenüber Isocyanaten, Isothiocyanaten und Lactimäthern"**

(73) Titulaire : **Société anonyme dite: LABORATOIRE ROGER BELLON**
**159, avenue A. Peretti**
**F-92201 Neuilly-sur-Seine (FR)**

(72) Inventeur : **Antoine, Michel**
**184, Rue Nationale**
**F-75013 Paris (FR)**
Inventeur : **Barreau, Michel**
**24bis, Avenue du Clos de Sénart**
**F-91230 Montgeron (FR)**
Inventeur : **Desconclois, Jean-Francois**
**12, Rue Beccaria**
**F-75012 Paris (FR)**
Inventeur : **Girard, Philippe**
**7, Rue du Bois-Gaudron Allainville**
**F-91290 Arpajon (FR)**
Inventeur : **Picaut, Guy**
**30, Rue Henri Cretté**
**F-94550 Chevilly Larue (FR)**

(74) Mandataire : **Savina, Jacques et al**
**RHONE-POULENC SANTE, Service Brevets,**
**20 Avenue Raymond Aron**
**F-92165 Antony Cédex (FR)**

**EP 0 379 413 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des dérivés de la benzo[b]naphtyridine-1,8 de formule générale :

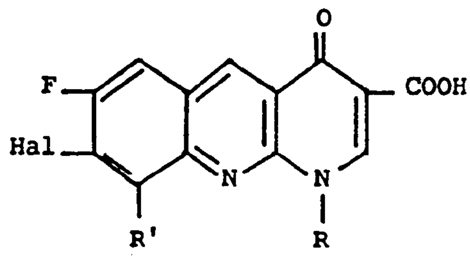

(I)

leurs sels, leur préparation et le cas échéant, les compositions qui les contiennent.

Dans les brevets US 4 229 456 et 4 133 885 ont été décrits des dérivés de naphtyridine de structure :

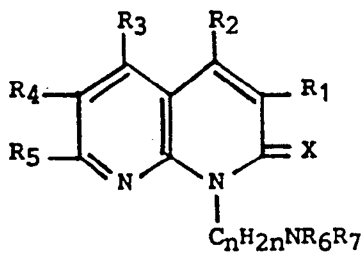

dans laquelle X peut être l'oxygène et deux des radicaux $R_1$ à $R_5$ adjacents peuvent former un cycle benzénique.

Ces produits sont utiles comme inhibiteurs des sécrétions gastriques acides.

La demande de brevet DE 3 302 126 décrit des hypotenseurs de formule générale :

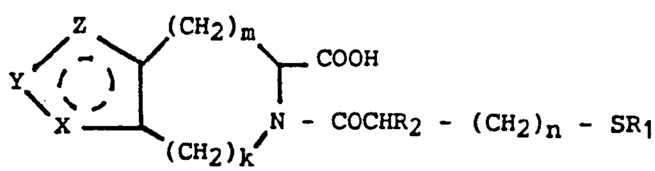

dans laquelle les radicaux X, Y et Z peuvent être O ou un radical $NR_4$ ou $CR_5=CR_5$ dans lequel les $R_5$ peuvent former un cycle benzénique.

Dans la formule générale (I)

- le symbole R représente un atome d'hydrogène, un radical alcoyle, fluoroalcoyle, cycloalcoyle contenant 3 à 6 atomes de carbone, alcoyloxy, alcoylamino ou représente un radical protecteur d'amine et,
- soit le symbole Hal représente un atome de fluor, de chlore ou de brome et le symbole R′ un atome d'hydrogène,
- soit les symboles Hal et R′ représentent simultanément des atomes de fluor.

Il est entendu que les radicaux alcoyle sont droits ou ramifiés et contiennent 1 à 4 atomes de carbone.

Lorsque R représente un radical protecteur, ce radical peut être tout groupement compatible et dont la mise en place et l'élimination n'altère pas le reste de la molécule. Notamment les groupements décrits par T.W. GREENE, Protective Groups in Organic Synthesis, A. Wiley-Interscience Publication (1981), ou par Mc OMIE, Protective Groups in Organic Chemistry, Plenum Press (1973).

A titre d'exemple, les groupements protecteurs peuvent être choisis parmi les radicaux triméthylsilyle, trityle, benzhydryle, tétrahydropyrannyle, formyle, acétyle, chloracétyle, trichloracétyle, trifluoracétyle, méthoxycarbonyle, éthoxycarbonyle, t.butoxycarbonyle, trichloréthoxycarbonyle, éthoxyméthyle, méthoxyméthyle.

Selon l'invention, le dérivé de la benzo[b]naphtyridine-1,8 de formule générale (I) peut être obtenu à partir de l'ester correspondant de formule générale :

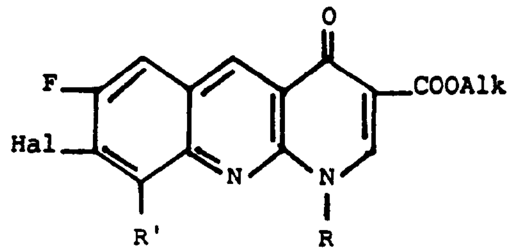

(II)

dans laquelle R est un atome d'hydrogène, un radical alcoyle, fluoroalcoyle, cycloalcoyle, alcoyloxy, alcoylamino ou alcoylamino protégé, Hal et R′ sont définis comme précédemment et Alk est un radical alcoyle contenant 1 à 4 atomes de carbone en chaîne droite ou ramifiée, par toute méthode connue pour obtenir un acide à partir d'un ester, sans toucher au reste de la molécule, suivie le cas échéant de la mise en place du radical protecteur R en position-1, ou de l'élimination du groupement protecteur du radical alcoylamino.

Lorsque R est un radical alcoylamino protégé, le radical protecteur peut être tout groupement protecteur d'amino compatible avec la molécule et les conditions opératoires du procédé. Notamment on utilise avantageusement des groupements qui peuvent être éliminés simultanément à l'hydrolyse de l'ester comme par exemple le radical formyle.

Généralement, la préparation de l'acide à partir de l'ester s'effectue par hydrolyse acide. On opère avantageusement dans un mélange acide acétique - acide chlorhydrique, dans l'acide sulfurique ou dans l'acide méthanesulfonique à une température comprise entre 60 et 100°C. Il est également possible d'opérer par saponification en présence de potasse ou de soude, en milieu hydroalcoolique, à une température comprise entre 20 et 80°C.

Le cas échéant la mise en place du radical protecteur en position-1 s'effectue selon les méthodes décrites dans les références ci-dessus.

L'ester dérivé de la benzo[b]naphtyridine-1,8 de formule générale (II) peut être préparé par action de l'amino-3 triazine-1,2,4 (pour obtenir un produit pour lequel R est un atome d'hydrogène) ou par action d'un produit de formule générale :

$$R - NH_2 \qquad (III)$$

dans laquelle R est alcoyle, fluoroalcoyle, cycloalcoyle, alcoyloxy, alcoylamino ou alcoylamino protégé, sur un dérivé de la quinoléine de formule générale :

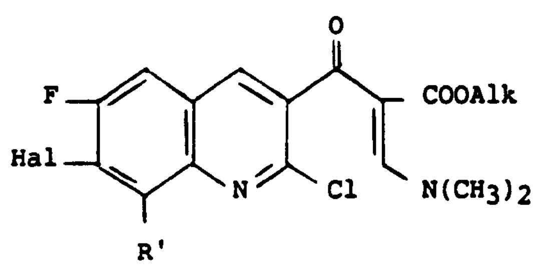

(IV)

dans laquelle R′, Hal et Alk sont définis comme précédemment, suivie de la cyclisation par action d'un agent accepteur d'acide.

Généralement, la réaction de l'amino-3 triazine-1,2,4 ou du produit de formule générale (III) est mise en oeuvre dans un solvant organique comme un alcool (éthanol, méthanol par exemple) ou un solvant chloré (trichlorométhane par exemple), à une température comprise entre 10 et 25°C.

La cyclisation s'effectue généralement en présence de diaza-1,8 bicyclo[5.4.0]undécène-7 ou d'une base azotée telle que par exemple la triéthylamine, ou un excès de l'amine employée dans un alcool à chaîne droite ou ramifiée contenant 1 à 4 atomes de carbone, à une température comprise entre 20°C et la température de reflux du mélange réactionnel.

Le dérivé de la quinoléine de formule générale (IV) peut être obtenu à partir du cétoester de formule générale :

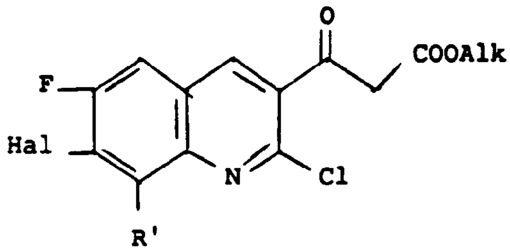

(V)

dans laquelle R′, Hal et Alk sont définis comme précédemment, par action d'un NN-diméthylformamide acétal de formule générale :

$$(CH_3)_2N - CH(OAlk_1)_2 \qquad (VI)$$

dans laquelle $Alk_1$ est un radical alcoyle contenant 1 à 4 atomes de carbone en chaîne droite ou ramifiée.

La réaction s'effectue généralement dans un solvant organique tel qu'un ester (acétate d'éthyle par exemple) à une température comprise entre 60 et 75°C.

Le cétoester de formule générale (V) pour lequel R′ est un atome d'hydrogène et Hal est défini comme précédemment peut être obtenu à partir de l'acide dichloro-2,7 fluoro-6 quinoléine carboxylique-3 ou de l'acide chloro-2 difluoro-6,7 quinoléine carboxylique-3 comme décrit ci-après aux exemples 1 et 6 ou à partir de l'acide bromo-7 chloro-2 fluoro-6 quinoléine carboxylique-3 par analogie avec cette méthode. Dans ce cas, la bromo-3 fluoro-4 aniline utilisée comme produit de départ, peut-être préparée selon la méthode décrite par W.B. Austin et coll., J. Org. Chem., 46(11), 2280 (1981).

Le cétoester de formule générale (V) pour lequel R′ et Hal sont simultanément des atomes de fluor, peut-être obtenu à partir de l'acide chloro-2 trifluoro-6,7,8 quinoléine carboxylique-3 comme décrit-après, à l'exemple 9.

Les nouveaux produits selon l'invention sont utiles comme intermédiaires pour la préparation de dérivés de la benzo[b]naphtyridine-1,8 de formule générale :

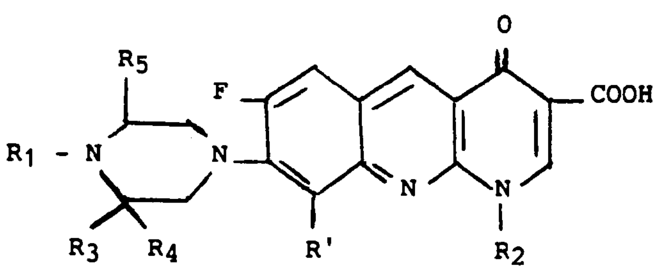

(VII)

dans laquelle :

- $R_1$ représente un atome d'hydrogène ou un radical alcoyle ou hydroxyalcoyle,
- $R_2$ représente un atome d'hydrogène ou un radical alcoyle, fluoroalcoyle, cycloalcoyle contenant 3 à 6 atomes de carbone, alcoyloxy ou alcoylamino,
- $R_3$ représente un radical alcoyle et
- $R_4$ et $R_5$ sont différents entre eux et représentent un atome d'hydrogène ou un radical alcoyle, ou bien
- $R_3$ est un atome d'hydrogène ou un radical alcoyle ou cycloalcoyle et
- $R_4$ et $R_5$ sont des atomes d'hydrogène,
- R′ représente atome d'hydrogène ou de fluor

ainsi que leurs sels, leurs hydrates et le cas échéant leurs isomères.

Les produits de formule générale (VII) peuvent être obtenus par substitution d'une pipérazine de formule générale :

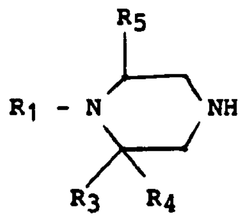

(VIII)

dans laquelle $R_1$, $R_3$, $R_4$ et $R_5$ sont définis comme précédemment, sur une benzo[b]naphtyridine-1,8 de formule générale (I), suivie éventuellement, si $R_1$ est un atome d'hydrogène et si l'on veut obtenir un dérivé de benzo[b]naphtyridine-1,8 dans laquelle $R_1$ est méthyle, de la transformation du produit obtenu en une (méthyl-4 pipérazinyl-1)-8 benzo[b]naphtyridine et suivie le cas échéant de l'élimination du radical protecteur en position 1.

L'action du dérivé de la pipérazine de formule générale (VIII) s'effectue généralement en présence d'un excès de ce dérivé comme accepteur d'acide ou en présence d'un accepteur d'acide organique ou minéral dans des solvants organiques convenables. Il est possible d'opérer avec ou sans solvant, à une température comprise entre 30 et 120°C. Lorsque l'on opère en présence d'un solvant, la réaction s'effectue avantageusement dans des solvants tels que la pyridine, le diméthylformamide, le diméthylsulfoxyde ou l'acétonitrile.

Il est entendu que, dans le cas où l'on veut obtenir un produit pour lequel le symbole $R_2$ est un atome d'hydrogène, il est préférable d'opérer à partir d'un dérivé de la benzonaphtyridine pour lequel R est un radical protecteur. La protection, et l'élimination du radical protecteur après la réaction, s'effectuent selon les méthodes habituelles.

Le cas échéant, l'opération subséquente de méthylation du radical pipérazinyle s'effectue avantageusement par action du formol en présence d'acide formique. On opère généralement en milieu aqueux, à une température comprise entre 90 et 100°C.

Les nouveaux produits selon la présente invention ainsi que les produits de formule générale (VII) peuvent être éventuellement purifiés par des méthodes physiques telles que la cristallisation ou la chromatographie.

Les produits selon la présente invention ainsi que les produits de formule générale (VII), peuvent être transformés en sels métalliques ou en sels d'addition avec les bases azotées selon les méthodes connues en soi. Ces sels peuvent être obtenus par action d'une base métallique (par exemple alcaline ou alcalinoterreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration, décantation ou lyophilisation.

Les dérivés de benzo[b]naphtyridine-1,8 de formule générale (VII) et leurs sels pharmaceutiquement acceptables présentent des propriétés antibactériennes. Ils manifestent une activité remarquable in vitro et in vivo sur les germes gram positifs et d'une manière générale sur les germes responsables de la plupart des infections des voies aériennes hautes et basses.

In vitro, les produits de formule générale (VII) se sont montrés actifs à une concentration comprise entre 0,12 et 50 µg/cm3 sur staphylococcus aureus IP 8203.

In vivo, les produits de formule générale (VII) se sont montrés actifs sur les infections expérimentales de la souris à staphylococcus aureus IP 8203 à des doses comprises entre 2 et 150 mg/kg par voie orale ou sous cutanée.

De plus ces produits sont faiblement toxiques. Leur $DL_{50}$ est généralement supérieure à 500 mg/kg par voie sous cutanée chez la souris.

Les nouveaux dérivés de benzo[b]naphtyridine de formule générale (I) pour lesquels R est autre qu'un radical protecteur, ainsi que leurs sels, sont aussi particulièrement intéressants pour leurs propres propriétés antibactériennes in vitro et peuvent être, de ce fait, particulièrement utiles pour un usage local dans le cas d'infections cutanées à staphylocoques.

Les dérivés de la benzo[b]naphtyridine-1,8 selon l'invention se sont, en effet, montrés actifs in vitro à des concentrations comprises entre 0,2 et 500 µg/cm3 sur staphylococcus aureus IP 8203.

Leur $DL_{50}$ est également supérieure à 500 mg/kg par voie sous cutanée chez la souris.

Comme exemples de sels pharmaceutiquement acceptables, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

D'un intérêt particulier sont les produits de formule générale (I) pour lesquels R représente un atome d'hydrogène, un radical alcoyle contenant 1 à 4 atomes de carbone, un radical fluoroéthyle, cyclopropyle, méthoxy ou méthylamino et soit Hal représente un atome de fluor ou de chlore et R' représente un atome d'hydrogène, soit Hal et R' représentent simultanément des atomes de fluor.

Et parmi ces produits, plus spécialement intéressants sont les produits suivants :

- acide difluoro-7,8 méthyl-1 oxo-4 dihydro-1,4 benzo[b]naphtyridine-1,8 carboxylique-3 ;
- acide chloro-8 fluoro-7 méthyl-1 oxo-4 dihydro-1,4 benzo[b]naphtyridine-1,8 carboxylique-3 ;
- acide méthyl-1 oxo-4 trifluoro-7,8,9 dihydro-1,4 benzo[b]naphtyridine-1,8 carboxylique-3 ;
- acide difluoro-7,8 méthoxy-1 oxo-4 dihydro-1,4 benzo[b]naphtyridine-1,8 carboxylique-3 ;
- acide cyclopropyl-1 difluoro-7,8 oxo-4 dihydro-1,4 benzo[b]naphtyridine-1,8 carboxylique-3 ;

Les exemples suivants donnés à titre non limitatif illustrent la présente invention.

## EXEMPLE 1

Une suspension de 15 g de chloro-8 éthoxycarbonyl-3 fluoro-7 méthyl-1 oxo-4 dihydro-1,4 benzo[b]naphtyridine-1,8 dans 150 cm3 d'acide acétique et 150 cm3 d'acide chlorhydrique en solution aqueuse à 17,5 % est chauffée à une température voisine de 100°C sous agitation pendant 4 heures. Après refroidissement à température voisine de 20°C, le produit est essoré, lavé par 2 fois 150 cm3 d'éthanol puis 2 fois 100 cm3 d'éther éthylique. On obtient 12,7 g d'acide chloro-8 fluoro-7 méthyl-1 oxo-4 dihydro-1,4 benzo[b]naphtyridine-1,8 carboxylique-3 sous forme d'un solide beige se sublimant à 400-405°C.

La chloro-8 éthoxycarbonyl-3 fluoro-7 méthyl-1 oxo-4 dihydro-1,4 benzo[b]naphtyridine-1,8 est préparée de la manière suivante :

Dans une suspension sous agitation de 19,3 g de (dichloro-2,7 fluoro-6 quinoléinecarbonyle-3)-2 diméthylamino-3 acrylate d'éthyle dans 250 cm3 d'éthanol, maintenue entre 10 et 15°C, on fait barboter de la méthylamine jusqu'à absorption de 16 g de gaz. On laisse la température remonter à environ 20°C, ajoute 0,8 g de diaza-1,8-bicyclo[5.4.0.]undécène-7 (DBU) et chauffe à une température voisine de 75°C pendant 2 heures. Après refroidissement à environ 20°C, le produit est essoré, lavé par 2 fois 150 cm3 d'éthanol et 2 fois 100 cm3 d'éther éthylique. On obtient 15 g de chloro-8 éthoxycarbonyl-3 fluoro-7 méthyl-1 oxo-4 dihydro-1,4 benzo[b]naphtyridine-1,8 sous forme d'un solide jaune fondant à 360-362°C qui est utilisé sans autre purification pour les étapes ultérieures.

Le (dichloro-2,7 fluoro-6 quinoléinecarbonyle-3)-2 diméthylamino-3 acrylate d'éthyle est préparé de la manière suivante :

Une suspension de 16,5 g de (dichloro-2,7 fluoro-6 quinolyl-3)-3 oxo-3 propionate d'éthyle dans 160 $cm^3$ d'acétate d'éthyle et 19 cm3 de N,N-diméthylformamide diméthylacétal est chauffée à une température voisine de 75°C, sous agitation, pendant 2 heures. Le mélange réactionnel est concentré à sec sous pression réduite (20 kPa) à 50°C. L'extrait sec est repris par 50 cm3 d'éther isopropylique, essoré, lavé par 2 fois 10 cm3 d'éther isopropylique. On obtient 16,57 g de (dichloro-2,7 fluoro-6 quinoléinecarbonyle-3)-2 diméthylamino-3 acrylate d'éthyle sous forme d'un solide orangé fondant à 122°C. Ce produit est utilisé sans autre purification pour les étapes ultérieures.

Le (dichloro-2,7 fluoro-6 quinolyl-3)-3 oxo-3 propionate d'éthyle est préparé de la manière suivante :

Une suspension de 38,75 g d'acide dichloro-2,7 fluoro-6 quinoléine carboxylique-3 dans 410 cm3 de trichlorométhane et 24 cm3 de chlorure de thionyle est chauffée à une température voisine de 60°C, sous agitation, pendant 6 heures. La solution obtenue est concentrée à sec sous pression réduite (20 kPa) à 50°C. L'extrait sec est repris 2 fois par au total 200 cm3 de toluène et concentré de nouveau sous pression réduite dans les mêmes conditions que précédemment. Le solide jaune obtenu fondant à 124°C est dissous dans 230 cm3 de tétrahydrofuranne anhydre. La solution obtenue est introduite, goutte à goutte, sous agitation, en 30 minutes, entre 5 et 10°C, dans 200 cm3 d'une solution de chélate de magnésium dans le tétrahydrofuranne dont la préparation est décrite ci-après. On laisse la température remonter à 20°C et agite 1 heure et demie à cette température. La solution obtenue est introduite, goutte à goutte, sous forte agitation à une température voisine de 5°C, dans 1 litre d'acide sulfurique 0,5N. On laisse la température de la suspension obtenue remonter à 20°C et agite encore 2 heures à cette température. On extrait par 1 litre d'acétate d'éthyle, filtre les phases organique et aqueuse sur silice diatomée pour filtration ce qui permet d'éliminer un léger insoluble, extrait la phase aqueuse encore 2 fois par 500 cm3 d'acétate d'éthyle. Les extraits organiques réunis sont lavés par 2 fois 500 cm3 d'eau, séchés sur sulfate de magnésium, filtrés et concentrés à sec sous pression réduite (20 kPa) à 40°C. Le résidu est repris par 100 cm3 d'éther isopropylique à 20°C, essoré et lavé par 2 fois 30 cm3 d'éther isopropylique. On obtient 40,55 g de (dichloro-2,7 fluoro-6 quinolyl-3)-3 oxo-3 propionate d'éthyle sous forme d'un solide beige fondant à 112-114°C. Ce produit est utilisé sans autre purification pour les étapes ultérieures.

Préparation du chélate de magnésium du monomalonate d'éthyle :

A 6,9 g de magnésium en tournure on ajoute progressivement 5 cm3 d'éthanol absolu, 0,2 cm3 de tétrachlorométhane et 2 g de monomalonate d'éthyle. Après échauffement, on ajoute, en 15 minutes, une solution de 23,8 g de monomalonate d'éthyle dans 450 cm3 d'éthanol. Le mélange est chauffé 20 heures à une température voisine de 78°C, concentré sous pression réduite (20 kPa) à 50°C. Le résidu est repris par 2 fois 100 cm3 de toluène et concentré sous pression réduite dans les mêmes conditions que précédemment. La poudre grise obtenue est mise en solution par addition de tétrahydrofuranne anhydre de façon à obtenir un volume total de 200 cm3.

Le monomalonate d'éthyle a été préparé selon la méthode décrite par D.S. Breslow, E. Baumgarten, C.R. Hauser., J. Am. Chem. Soc., 66, 1287 (1944) et distillé sous pression réduite (point d'ébullition=132°C/2,7 kPa).

L'acide dichloro-2,7 fluoro-6 quinoléine carboxylique-3 est préparé de la manière suivante :

A une suspension, sous agitation, refroidie à 10°C, de 69,5 g de dichloro-2,7 fluoro-6 formyl-3 dihydro-1,4 quinoléine dans 282 cm3 de potasse aqueuse 2N et 282 cm3 d'eau, on ajoute en 1 heure en maintenant la température entre 10 et 14°C, une solution de 89,3 g de permanganate de potassium dans 1,4 litre d'eau. On laisse la température remonter à environ 20°C et agite encore 30 minutes à cette température. On ajoute 26 g de dithionite de sodium, agite 10 minutes à température voisine de 20°C, filtre sur silice diatomée pour filtration, lave par 2 fois 250 cm3 d'eau. Le filtrat et les phases aqueuses de lavage sont réunis et additionnés de 90 cm3 d'acide chlorhydrique en solution aqueuse à 35 %. Le précipité formé est extrait par 4 fois 500 cm3 d'acétate d'éthyle. Les extraits organiques réunis sont lavés par 3 fois 500 cm3 d'eau, séchés sur sulfate de magnésium, filtrés et concentrés sous pression réduite (20 kPa) à 50°C. Le résidu est repris par 350 cm3 d'éther éthylique, essoré, lavé par 2 fois 200 cm3 d'éther éthylique. On obtient 45 g d'acide dichloro-2,7 fluoro-6 quinoléine carboxylique-3 sous forme d'un solide beige fondant à 230°C qui est utilisé sans autre purification pour les étapes ultérieures.

La dichloro-2,7 fluoro-6 formyl-3 dihydro-1,4 quinoléine a été préparée de la manière suivante :

A un mélange de 250 cm3 de trichlorométhane et de 54 cm3 de diméthylformamide, on ajoute en 30 minutes, sous agitation, entre 10 et 15°C, 55,6 cm3 de chlorure de phosphoryle et agite 1 heure à température voisine de 20°C. A la solution obtenue, on ajoute en 10 minutes, à environ 20°C, progressivement, sous forte agitation, 52 g de chloro-7 fluoro-6 dihydro-3,4 carbostyrile. La suspension obtenue est chauffée à une température voisine de 60°C et maintenue encore 2 heures, sous agitation à cette température. Le mélange réactionnel est concentré sous pression réduite (20 kPa) à 50°C jusqu'à obtention d'un mélange pâteux. Sous forte agitation, on ajoute un mélange de 250 cm3 d'eau et 250 g de glace pilée. Le solide obtenu est essoré à environ 5°C et lavé par 4 fois 125 cm3 d'eau à 5°C. Le produit humide obtenu et 58 g d'acétate de sodium sont ajoutés simultanément, en 1 heure, à 500 cm3 d'eau à 90°C de manière à maintenir le pH à environ 6. On agite encore 15 minutes à 90°C, laisse la température redescendre à environ 50°C, essore à cette température et lave par 3 fois 250 cm3 d'eau à environ 20°C. On obtient 54,3 g de dichloro-2,7 fluoro-6 formyl-3 dihydro-1,4 quinoléine sous forme d'un solide jaune fondant à 260°C qui est utilisé tel quel pour les étapes ultérieures.

Le chloro-7 fluoro-6 dihydro-3,4 carbostyrile est préparé de la manière suivante :

A 174,4 g de chloro-3′ fluoro-4′ N-chloro-3 propionanilide, on ajoute sous forte agitation en 5 minutes, 350 g de chlorure d'aluminium. Le mélange solide est chauffé en 30 minutes à environ 60°C. La température monte d'elle-même à environ 80°C et le mélange réactionnel devient liquide. On chauffe alors à 110°C en 15 minutes et maintient entre 110 et 120°C pendant 3 heures. Le mélange réactionnel (à environ 110°C) est versé, en 10 minutes, sous forte agitation, dans un mélange de 550 cm3 d'acide chlorhydrique à 35 % et de 500 g de glace pilée. On laisse la température remonter à 20°C, essore, lave par 6 fois 500 cm3 d'eau.

Le produit humide est recristallisé dans 1,2 litre d'éthanol. On obtient 108 g de chloro-7 fluoro-6 dihydro-3,4 carbostyrile sous forme d'un solide beige fondant à 215°C.

La chloro-3′ fluoro-4′ N-chloro-3 propionanilide a été préparée de la manière suivante :

A une solution, à température voisine de 55°C, de 291 g de chloro-3 fluoro-4 aniline dans 500 cm3 d'acétone, on ajoute, sous agitation, en 35 minutes, une solution de 127 g de chlorure de l'acide chloro-3 propionique dans 200 cm3 d'acétone et maintient à cette température pendant 2 heures. Après refroidissement à environ 20°C, l'insoluble est éliminé par filtration et lavé par 2 fois 200 cm3 d'acétone. Le filtrat et les lavages réunis sont versés sous agitation dans 2 litres d'eau et 1 kg de glace. On laisse la température remonter à environ 20°C, extrait par 4 fois 500 cm3 de dichlorométhane. Les extraits organiques réunis sont lavés par 3 fois 500 cm3 d'eau, séchés sur sulfate de magnésium, agités 15 minutes avec 6 g de charbon végétal Norit, filtrés sur silice diatomée pour filtration et concentrés sous pression réduite (20 kPa) à 50°C. Le solide obtenu est recristallisé dans un mélange de 133 cm3 de cyclohexane et de 67 cm3 d'éther isopropylique. On obtient 176 g de chloro-3′fluoro-4′ N-chloro-3 propionanilide sous forme d'un solide beige fondant à 94°C qui peut être utilisé tel quel pour les étapes ultérieures.

EXEMPLE 2

En opérant dans les conditions décrites à l'exemple 1, mais à partir de 10,5 g de chloro-8 fluoro-7 éthoxycarbonyl-3 éthyl-1 oxo-4 dihydro-1,4 benzo[b]naphtyridine-1,8. On obtient 9,3 g d'acide chloro-8 éthyl-1 fluoro-7 oxo-4 dihydro-1,4 benzo[b]naphtyridine-1,8 carboxylique-3 sous forme d'un solide beige fondant à 380°C.

La chloro-8 éthoxycarbonyl-3 éthyl-1 fluoro-7 oxo-4 dihydro-1,4 benzo[b]naphtyridine-1,8 est préparée de la manière suivante :

Dans une suspension sous agitation de 13,5 g de (dichloro-2,7 fluoro-6 quinoléinecarbonyle-3)-2 diméthylamino-3 acrylate d'éthyle dans 135 cm3 d'éthanol, on ajoute, en 5 minutes, entre 10 et 15°C, 16 g d'éthylamine, laisse la température remonter à environ 20°C, ajoute 0,5 g de DBU et chauffe sous agitation, pendant 2 heures, à une température voisine de 75°C. Après refroidissement à une température voisine de 20°C, le précipité est essoré, lavé par 2 fois 100 cm3 d'éthanol, 2 fois 100 cm3 d'éther éthylique. On obtient 10,4 g de chloro-8 éthoxycarbonyl-3 éthyl-1 fluoro-7 oxo-4 dihydro-1,4 benzo[b]naphtyridine-1,8 sous forme d'un solide jaune fondant à 300-301°C, qui peut être utilisé sans autre purification pour les étapes ultérieures.

EXEMPLE 3

Une suspension de 16,4 g de chloro-8 éthoxycarbonyl-3 fluoro-7 N-formyl N-méthyl amino-1 oxo-4 dihydro- 1,4 benzo[b]naphtyridine-1,8 dans 164 cm3 d'acide acétique et 164 cm3 d'acide chlorhydrique en solution aqueuse à 17,5 % est chauffée à une température voisine de 100°C, sous agitation pendant 4 heures. Après refroidissement à température voisine de 10°C, on ajoute entre 10 et 20°C, 165 cm3 de lessive de soude à 30 %. Le produit est essoré, lavé par 3 fois 150 cm3 d'eau, 3 fois 150 cm3 d'éthanol et 3 fois 150 cm3 d'éther éthylique. On obtient 13,64 g d'acide chloro-8 fluoro-7 méthylamino-1 oxo-4 dihydro-1,4 benzo[b]naphtyridine-1,8 carboxylique-3 sous forme d'un solide jaune fondant à 354-356°C.

La chloro-8 éthoxycarbonyl-3 fluoro-7 (N-formyl N-méthyl amino)-1 oxo-4 dihydro-1,4 benzo[b]naphtyridine-1,8 est préparée dans les conditions de l'exemple 1 mais à partir de 19,25 g de (dichloro-2,7 fluoro-6 quinoléinecarbonyle-3)-2 diméthylamino-3 acrylate d'éthyle, de 4,05 g de N-formyl N-méthyl hydrazine et de 1,6 g de DBU, dans 200 cm3 d'éthanol. On obtient 16,4 g de chloro-8 éthoxycarbonyl-3 fluoro-7 (N-formyl N-méthyl amino)-1 oxo-4 dihydro- 1,4 benzo[b]naphtyridine-1,8 sous forme de solide incolore fondant à 296-298°C, qui peut être utilisé sans autre purification pour les étapes ultérieures.

La N-formyl-N-méthylhydrazine peut être préparée selon la méthode décrite par Carl Th. Pedersen, Acta Chem. Scand., 18(9), 2199 (1964).

EXEMPLE 4

En opérant dans les conditions décrites à l'exemple 1, mais à partir de 6,1 g de chloro-8 cyclopropyl-1 éthoxycarbonyl-3 fluoro-7 oxo-4 dihydro-1,4 benzo[b]naphtyridine-1,8. On obtient 4,85 g d'acide chloro-8 cyclopropyl-1 fluoro-7 oxo-4 dihydro-1,4 benzo[b]- naphtyridine-1,8 carboxylique-3 sous forme d'un solide jaune fondant à 330°C.

La chloro-8 cyclopropyl-1 éthoxycarbonyl-3 fluoro-7 oxo-4 dihydro-1,4 benzo[b]naphtyridine-1,8 est préparée dans les conditions suivantes :

Une solution de 20,6 g de (dichloro-2,7 fluoro-6 quinoléinecarbonyle-3)-2 diméthylamino-3 acrylate d'éthyle et de 6 g de cyclopropylamine dans 100 cm3 de trichlorométhane est agitée à une température voisine de 20°C pendant 24 heures. Le mélange réactionnel est concentré sous pression réduite (20 kPa) à 50°C. Le résidu est repris par 180 cm3 d'éthanol et 10 g de DBU et la solution obtenue chauffée à une température voisine de 78°C pendant 4 heures. Après refroidissement à une température voisine de 20°C, le précipité obtenu est essoré et lavé par 2 fois 60 cm3 d'éthanol. On obtient 13,65 g de chloro-8 cyclopropyl-1 éthoxycarbonyl-3 fluoro-7 oxo-4 dihydro-1,4 benzo[b]naphtyridine-1,8 sous forme d'un solide jaune pâle fondant à 256°C qui est utilisé sans autre purification pour les étapes ultérieures.

EXEMPLE 5

Une suspension de 1,88 g de chloro-8 éthoxycarbonyl-3 fluoro-7 oxo-4 tertiobutyl-1 dihydro-1,4 benzo[b]naphtyridine-1,8 dans 10 cm3 d'éthanol, 5 cm3 d'eau et 15 cm3 de potasse aqueuse 2N est chauffée à une température voisine de 75°C, sous agitation pendant une heure. La solution obtenue est additionnée de 2 cm3 d'acide acétique. Le précipité formé est essoré, lavé par 3 fois 10 cm3 d'eau, 3 fois 10 cm3 d'éthanol. Après une recristallisation dans 50 cm3 de diméthylformamide, on obtient 1,7 g d'acide chloro-8 fluoro-7 oxo-4 tertiobutyl-1 dihydro-1,4 benzo[b]naphtyridine-1,8 carboxylique-3 sous forme d'un solide jaune fondant à

398°C.

La chloro-8 éthoxycarbonyl-3 fluoro-7 oxo-4 tertiobutyl-1 dihydro-1,4 benzo[b]naphtyridine-1,8 est préparée dans les conditions de l'exemple 4, mais à partir de 8,86 g (dichloro-2,7 fluoro-6 quinoléinecarbonyle-3)-2 diméthylamino-3 acrylate d'éthyle, de 4,03 g de tertiobutylamine dans 45 cm3 de trichlorométhane, puis dans 4,53 g de DBU et 45 cm3 d'éthanol. On obtient 5 g de chloro-8 éthoxycarbonyl-3 fluoro-7 oxo-4 tertiobutyl-1 dihydro-1,4 benzo[b]naphtyridine-1,8 sous forme d'un solide jaune fondant à 239°C.

EXEMPLE 6

Une suspension de 1,95 g de cyclopropyl-1 éthoxycarbonyl-3 difluoro-7,8 oxo-4 dihydro-1,4 benzo [b] naphtyridine-1,8 dans 20 cm3 d'acide chlorhydrique à 17,5 % et 20 cm3 d'acide acétique est chauffée à une température voisine de 100°C pendant 1 heure 30 minutes. Après refroidissement à environ 20°C, le mélange réactionnel est versé dans 100 cm3 d'eau. Le précipité est essoré, lavé par par 3 fois 20 cm3 d'eau. Après 1 recristallisation dans un mélange de 30 cm3 de diméthylformamide et de 30 cm3 d'éthanol on obtient 1,31 g d'acide cyclopropyl-1 difluoro-7,8 oxo-4 dihydro-1,4 benzo[b]naphtyridine-1,8 carboxylique-3 sous forme d'un solide jaune fondant à 284-285°C.

Le cyclopropyl-1 éthoxycarbonyl-3 difluoro-7,8 oxo-4 dihydro-1,4 benzo[b]naphtyridine-1,8 est préparé de la manière suivante :

Une suspension sous agitation de 5,27 g de (chloro-2 difluoro-6,7 quinoléinecarbonyle-3)-2 cyclopropylamino-3 acrylate d'éthyle, dans 2,22 g de diaza-1,8 bicyclo [5,4,0] undécène-7 (DBU) et 120 cm3 d'éthanol est chauffée à une température voisine de 75°C pendant 35 minutes. Après refroidissement à environ 20°C, le mélange réactionnel est repris par 100 cm3 d'eau, extrait par 1 fois 100 cm3 et 2 fois 50 cm3 de trichlorométhane. Les extraits organiques réunis sont lavés par 3 fois 50 cm3 d'eau, séchés sur sulfate de magnésium, filtrés et concentrés sous pression réduite (20 kPa) à une température voisine de 20°C. L'extait sec obtenu est repris par 30 cm3 d'éther isopropylique éssoré et recristallisé dans un mélange de 75 cm3 d'éthanol et de 75 cm3 de diméthylformamide. On obtient 3,57 g de cyclopropyl-1 éthoxycarbonyl-3 difluoro-7,8 oxo-4 dihydro-1,4 benzo[b]naphtyridine-1,8 sous forme d'un solide jaune fondant à 229-230°C.

Le (chloro-2 difluoro-6,7 quinoléinecarbonyle-3)-2 cyclopropylamino-3 acrylate d'éthyle est préparé de la manière suivante :

Une solution de 6,25 g de (chloro-2 difluoro-6,7 quinoléinecarbonyle-3)-2 diméthylamino-3 acrylate d'éthyle dans 2,91 g de cyclopropylamine et 25 cm3 de trichlorométhane est agitée pendant 3 heures à une température voisine de 20°C. Le mélange réactionnel est concentré sous pression réduite (20 kPa) à une température voisine de 50°C. L'extrait sec est repris par 50 cm3 d'éther isopropylique, essoré, puis lavé par 20 cm3 du même solvant.

On obtient 5,27 g de (chloro-2 difluoro-6,7 quinoléinecarbonyle-3)-2 cyclopropylamino-3 acrylate d'éthyle sous forme d'un solide orangé fondant à 116-117°C. Ce produit est utilisé sans autre purification pour les étapes ultérieures.

Le (chloro-2 difluoro-6,7 quinoléinecarbonyle-3)-2 diméthylamino-3 acrylate d'éthyle est préparé de la manière suivante :

Une suspension de 6,17 g de (chloro-2 difluoro-6,7 quinolyl-3)-oxo-3 propionate d'éthyle dans 7,15 g de N,N-diméthylformamidediméthylacétal et 60 cm3 d'acétate d'éthyle est chauffée à une température voisine de 75°C pendant 1 heure 15 minutes. Le mélange réactionnel est concentré à sec sous pression réduite (20 kPa) à environ 50°C. Le résidu est repris par 50 cm3 d'éther isopropylique, essoré, lavé par 3 fois 25 cm3 du même solvant. On obtient 6,65 g de (chloro-2 difluoro-6,7 quinoléinecarbonyle-3)-2 diméthylamino-3 acrylate d'éthyle sous forme d'un solide orangé fondant à 140°C.

Le (chloro-2 difluoro-6,7 quinolyl-3)-3 oxo-3 propionate d'éthyle est préparé de la manière suivante :

Une suspension de 14,13 g d'acide chloro-2 difluoro-6,7 quinoléine carboxylique-3 dans 29 cm3 de chlorure de thionyle et 220 cm3 de trichlorométhane est chauffée à une température voisine de 60°C pendant 4 heures. La solution obtenue est concentrée à sec sous pression réduite (20 kPa) à environ 60°C. Le résidu obtenu est repris par 75 cm3 de n-hexane, essoré, lavé par 2 fois 60 cm3 du même solvant. Les 14,4 g du solide jaune obtenu sont mis en solution dans 115 cm3 de tétrahydrofuranne. Cette solution est introduite, goutte à goutte, sous agitation, en 35 minutes, entre 5 et 10°C, dans 70 cm3 d'une solution de chélate de magnésium du monomalonate d'éthyle dans le tétrahydrofuranne préparé dans les conditions décrites ci-après. On laisse la température remonter à environ 20°C, et agite encore 2 heures dans ces conditions. La solution obtenue est introduite, goutte à goutte, sous agitation, en 30 minutes, à une température voisine de 5°C, dans 560 cm3 d'acide sulfurique 0,5 N. On laisse la température de la suspension obtenue remonter à 20°C et agite encore 1 heure et demie à cette température. On extrait par 3 fois 250 cm3 d'acétate d'éthyle. Les extraits organiques réunis sont lavés par 2 fois 250 cm3 d'eau, séchés sur sulfate de magnésium, filtrés, et concentrés à sec, sous

pression réduite (20 kPa) à 50°C. Le résidu obtenu est repris par 50 cm3 de n-hexane à 20 % d'éther isopropylique, essoré, lavé par 10 cm3 du même mélange et recristallisé dans 60 cm3 d'isopropanol à 30 % de n-hexane. On obtient 11,84 g de (chloro-2 difluoro-6,7 quinolyl-3)-3 oxo-3 propionate d'éthyle sous forme d'un solide crème fondant à 107°C.

Préparation du chélate de magnésium du monomalonate d'éthyle :

A 2,78 g de magnésium en tournure on ajoute progressivement 2 cm3 d'éthanol absolu, 0,1 cm3 de tétrachlorométhane et 1 g de monomalonate d'éthyle. Après échauffement, on ajoute, en 15 minutes, une solution de 9 g de monomalonate d'éthyle dans 180 cm3 d'éthanol. Le mélange est chauffé 20 heures à une température voisine de 75°C, concentré sous pression réduite (20 kPa) à 50°C. Le résidu est repris par 2 fois 100 cm3 de toluène et concentré sous pression réduite dans les mêmes conditions que précédemment. La poudre grise obtenue est mise en solution par addition de tétrahydrofuranne anhydre de façon à obtenir un volume total de 70 cm3.

L'acide chloro-2 difluoro-6,7 quinoléine carboxylique-3 a été préparé de la manière suivante :

A une suspension, sous agitation, refroidie à 10°C, de 70,18 g de chloro-2 difluoro-6,7 formyl-3 dihydro-1,4 quinoléine dans 970 cm3 de potasse aqueuse N, on ajoute, en 1 heure, en maintenant la température entre 10 et 14°C, une solution de 115 g de permanganate de potassium dans 1,215 litre d'eau. On laisse la température remonter à environ 20°C et agite encore 30 minutes à cette température. On ajoute 38,5 g de dithionite de sodium, agite 10 minutes à une température voisine de 20°C, filtre sur silice diatomée, lave par 3 fois 200 cm3 d'eau. Le filtrat et les phases aqueuses de lavage sont réunis et additionnés de 140 cm3 d'acide chlorhydrique en solution aqueuse à 35 %. Le précipité formé est extrait par 4 fois 800 cm3 d'acétate d'éthyle. Les extraits organiques réunis sont lavés par 2 fois 500 cm3 d'eau, séchés sur sulfate de magnésium, filtrés et concentrés sous pression réduite (20 kPa) à 50°C. Le résidu est repris par 400 cm3 d'éther éthylique, essoré, lavé par 2 fois 200 cm3 du même solvant. On obtient 49,2 g d'acide chloro-2 difluoro-6,7 quinoléine carboxylique-3 sous forme d'un solide beige fondant à 232°C qui est utilisé, sans autre purification, pour les étapes ultérieures.

La chloro-2 difluoro-6,7 formyl-3 dihydro-1,4 quinoléine a été préparée de la manière suivante :

A un mélange de 800 cm3 de trichlorométhane et de 74,35 cm3 de diméthylformamide, on ajoute en 30 minutes, sous agitation, entre 10 et 15°C, 76,9 cm3 de chlorure de phosphoryle et agite 1 heure à température voisine de 20°C. A la solution obtenue, on ajoute en 10 minutes à environ 20°C, sous forte agitation, 65,8 g de difluoro-6,7 dihydro-3,4 carbostyrile. La suspension obtenue est chauffée à une température voisine de 60°C et maintenue à cette température pendant 2 heures. Le mélange réactionnel est concentré sous pression réduite (20 kPa) à 50°C jusqu'à obtention d'un mélange pâteux. Sous forte agitation on ajoute un mélange de 500 g de glace et 500 cm3 d'eau. Le solide obtenu est essoré à environ 5°C et lavé par 3 fois 300 cm3 d'eau à 5°C. Le produit humide obtenu et 60 g d'acétate de sodium sont ajoutés simultanément, en 1 heure, à 1,5 litre d'eau à 90°C, de manière à maintenir le pH à environ 6. On agite encore 30 minutes à 90°C, laisse la température redescendre à environ 50°C, essore à cette température et lave par 3 fois 300 cm3 d'eau à environ 20°C. On obtient 70,18 g de chloro-2 difluoro-6,7 formyl-3 dihydro-1,4 quinoléine sous forme d'un solide jaune fondant à 260°C qui est utilisé tel quel pour les étapes ultérieures.

Le difluoro-6,7 dihydro-3,4 carbostyrile est obtenu de la manière suivante :

A 67 g de difluoro-3′,4′ N-chloro-3 propionanilide, on ajoute sous forte agitation, 134 g de chlorure d'aluminium, puis après environ 2 minutes, on ajoute, de nouveau, par petites fractions, en 15 minutes, 135,9 g de difluoro-3′,4′ N-chloro-3 propionanilide et 272 g de chlorure d'aluminium. La température monte d'elle-même à environ 60°C et le mélange réactionnel devient liquide. On chauffe alors à 110°C en 20 minutes et maintient entre 110 et 120°C pendant 2 heures. Le mélange réactionnel (à environ 110°C) est versé, en 10 minutes, sous forte agitation dans un mélange de 840 cm3 d'acide chlorhydrique à 35 % et de 1 kg de glace pilée. On laisse la température remonter à environ 20°C, essore, lave par 600 cm3 d'eau 2 fois, 300 cm3 d'éthanol à 5°C et 2 fois 400 cm3 d'éther éthylique à environ 20°C. On obtient 131,58 g de difluoro-6,7 dihydro-3,4 carbostyrile sous forme d'un solide beige fondant à 216°C qui est utilisé tel quel pour les étapes ultérieures.

Le difluoro-3′,4′ N-chloro-3 propionanilide est préparé de la manière suivante :

A une solution de 125 g de 3,4 difluoroaniline dans 80 cm3 de pyridine et 1,5 litre d'acétone chauffée à une température voisine de 55°C, on ajoute, sous agitation, en 1 heure et demie, 139,16 g de chlorure de l'acide chloro-3 propionique et maintient à cette température pendant 1 heure et demie. Après refroidissement à environ 20°C, la solution est versée, sous agitation, dans un mélange de 1 litre d'eau et 500 g de glace pilée. On laisse la température remonter à environ 20°C et extrait par 3 fois 500 cm3 de dichlorométhane. Les extraits organiques réunis sont lavés par 500 cm3 d'acide chlorhydrique N, 5 fois 500 cm3 d'eau, séchés sur sulfate de magnésium, filtrés et concentrés sous pression réduite (20 kPa) à environ 50°C. Le solide obtenu est repris par 500 cm3 de n-hexane, essoré, lavé par 2 fois 100 cm3 du même solvant. On obtient 202,9 g de difluoro-3′,4′ N-chloro-3 propionanilide sous forme d'un solide beige fondant à 76°C qui est utilisé sans autre purification pour les étapes ultérieures.

EXEMPLE 7

Une suspension de 2,78 g d'éthoxycarbonyl-3 difluoro-7,8 méthoxy-1 oxo-4 dihydro-1,4 benzo [b] naphtyridine-1,8 dans 30 cm3 d'acide chlorhydrique à 17,5 % et 30 cm3 d'acide acétique est chauffée à une température voisine de 100°C pendant 1 heure. Après refroidissement à environ 20°C, le mélange réactionnel est versé dans 100 cm3 d'eau. Le précipité formé est essoré, lavé par 3 fois 30 cm3 d'eau et 2 fois 5 cm3 d'éthanol. Après recristallisation dans 100 cm3 de diméthylformamide à 20 % d'éthanol, on obtient 2,03 g d'acide difluoro-7,8 méthoxy-1 oxo-4 dihydro-1,4 benzo [b] naphtyridine-1,8 carboxylique-3 sous forme d'un solide jaune fondant à 325-327°C.

L'éthoxycarbonyl-3 difluoro-7,8 méthoxy-1 oxo-4 dihydro-1,4 benzo [b] naphtyridine-1,8 est préparé dans les conditions suivantes :

Une suspension de 1,7 g de chlorhydrate de méthoxylamine dans 40 cm3 de trichlorométhane est additionnée de 2,13 g de triéthylamine. Après 15 minutes d'agitation à une température voisine de 20°C, la solution obtenue est additionnée de 3,69 g (chloro-2 difluoro-6,7 quinoléinecarbonyle-3)-2 diméthylamino-3 acrylate d'éthyle et agitée pendant 4 heures et demie à environ 20°C. Le mélange réactionnel est concentré à sec sous pression réduite (20 kPa) à une température voisine de 50°C. Le résidu est repris par 70 cm3 d'éthanol, 3,6 g de triéthylamine et chauffé pendant 30 minutes à une température voisine de 75°C. Après refroidissement à environ 20°C, le précipité obtenu est essoré et lavé par 3 fois 30 cm3 d'éthanol. On obtient 2,67 g d'éthoxycarbonyl-3 difluoro-7,8 méthoxy-1 oxo-4 dihydro-1,4 benzo [b] naphtyridine-1,8 sous forme d'un solide jaune pâle fondant à 266-268°C.

EXEMPLE 8

Une suspension de 8 g d'éthoxycarbonyl-3 difluoro-7,8 méthyl-1 oxo-4 dihydro-1,4 benzo [b] naphtyridine-1,8 dans 80 cm3 d'acide chlorhydrique en solution aqueuse à 17,5 % et 80 cm3 d'acide acétique est chauffée, sous agitation, à une température voisine de 100°C pendant 1 heure et demie. Après refroidissement à environ 20°C, le solide est essoré et lavé par 6 fois 100 cm3 d'eau. Après 1 recristallisation dans 160 cm3 de diméthylformamide, on obtient 6,44 g d'acide difluoro-7,8 méthyl-1 oxo-4 dihydro-1,4 benzo [b] naphtyridine-1,8 carboxylique-3, sous forme d'un solide jaune se décomposant à 360°C.

L'éthoxycarbonyl-3 difluoro-7,8 méthyl-1 oxo-4 dihydro-1,4 benzo [b] naphtyridine-1,8 est préparée de la manière suivante :

Dans une suspension sous agitation de 22,3 g de (chloro-2 difluoro-6,7 quinoléinecarbonyle-3)-2 diméthylamino-3 acrylate d'éthyle dans 480 cm3 d'éthanol, maintenue à une température voisine de 0°C, on ajoute en 10 minutes entre 0 et 5°C, une solution de 11,3 g à environ 0°C de méthylamine dans 50 cm3 d'éthanol, agite 1 heure entre 0 et 5°C, laisse la température remonter à environ 25°C et agite encore 16 heures à cette même température. L'insoluble est essoré, lavé par 3 fois 100 cm3 d'éthanol et 2 fois 100 cm3 d'éther éthylique. Après 1 recristallisation dans 250 cm3 de diméthylformamide, on obtient 16 g d'éthoxycarbonyl-3 difluoro-7,8 méthyl-1 oxo-4 dihydro-1,4 benzo [b] naphtyridine-1,8 sous forme d'un solide jaune fondant à 323-324°C.

EXEMPLE 9

Une suspension de 4 g d'éthoxycarbonyl-3 trifluoro-7,8,9 méthyl-1 oxo-4 dihydro-1,4 benzo [b] naphtyridine-1,8 dans 30 cm3 d'acide acétique et 30 cm3 d'acide chlorhydrique à 50 % est chauffée à une température voisine de 100°C pendant 2 heures. Après refroidissement à environ 20°C, on ajoute 100 cm3 d'eau. Le précipité formé est essoré, lavé par 3 fois 50 cm3 d'eau et recristallisé dans 80 cm3 de diméthylformamide. On obtient 3,4 g d'acide trifluoro-7,8,9 méthyl-1 oxo-4 dihydro-1,4 benzo[b]naphtyridine-1,8 carboxylique-3 sous forme d'un solide incolore fondant à 350-352°C.

L'éthoxycarbonyl-3 trifluoro-7,8,9 méthyl-1 oxo-4 dihydro-1,4 benzo [b] naphtyridine-1,8 est préparé de la manière suivante :

Dans une suspension sous agitation de 19,3 g de (chloro-2 trifluoro-6,7,8 quinoléinecarbonyle-3)-2 diméthylamino-3 acrylate d'éthyle dans 150 cm3 d'éthanol maintenue à une température voisine de 5°C, on ajoute en 10 minutes entre 5 et 10°C, une solution à environ 5°C de 10 g de méthylamine dans 50 cm3 d'éthanol, agite 1 heure entre 5 et 10°C et laisse la température remonter à environ 20°C. La solution obtenue est additionnée de 7,6 g de D.B.U. et chauffée à environ 30°C pendant 1 heure. Après refroidissement à une température voisine de 20°C, le produit est essoré, lavé par 2 fois 100 cm3 d'éthanol et 2 fois 100 cm3 d'éther isopropylique. On obtient 13,4 g d'éthoxycarbonyl-3 trifluoro-7,8,9 méthyl-1 oxo-4 dihydro-1,4 benzo [b] naphtyridine-1,8 sous forme d'un solide jaune fondant à 320°C qui est utilisé sans autre purification pour les étapes ultérieures.

Le (chloro-2 trifluoro-6,7,8 quinoléinecarbonyle-3)-2 diméthylamino-3 acrylate d'éthyle peut être préparé de la manière suivante :

Une suspension de 26,7 g de (chloro-2 trifluoro-6,7,8 quinolyl-3)-3 oxo-3 propionate d'éthyle dans 270 cm3 d'acétate d'éthyle et 32 cm3 de N,N diméthylformamidediméthylacétal est chauffée à une température voisine de 75°C, sous agitation, pendant 2 heures. Le mélange réactionnel est concentré à sec sous pression réduite (20kPa) à environ 50°C. L'extrait sec est repris par 175 cm3 d'éther isopropylique, essoré, lavé par 2 fois 85 cm3 du même solvant. On obtient 19,32 g de (chloro-2 trifluoro-6,7,8 quinoléinecarbonyle-3)-2 diméthylamino-3 acrylate d'éthyle sous forme d'un solide orange fondant à 118°C qui est utilisé sans autre purification pour les étapes ultérieures.

Le (chloro-2 trifluoro-6,7,8 quinolyl-3)-3 oxo-3 propionate d'éthyle est préparé de la manière suivante :

Une suspension de 46,3 g d'acide chloro-2 trifluoro-6,7,8 quinoléine carboxylique-3 dans 640 cm3 de trichlorométhane et 84 cm3 de chlorure de thionyle est chauffée, sous agitation, à une température voisine de 60°C pendant 6 heures. La solution obtenue est concentrée à sec sous pression réduite (20kPa) à environ 50°C. L'extrait sec obtenu est repris par 140 cm3 d'éther de pétrole (40-60), essoré, lavé par 2 fois 60 cm3 du même solvant. Les 47,61 g du solide jaune obtenu sont mis en solution dans 400 cm3 de tétrahydrofuranne. Cette solution est introduite, goutte à goutte, sous agitation, en 1 heure et demie, entre 5 et 10°C, dans 250 cm3 d'une solution de chélate de magnésium du monomalonate d'éthyle dans le tétrahydrofuranne préparé dans les conditions de l'exemple 6. On laisse la température remonter à environ 20°C et agite encore 2 heures dans ces conditions. La solution obtenue, est introduite, goutte à goutte, sous forte agitation, en 1 heure, à une température voisine de 5°C dans 1750 cm3 d'acide sulfurique 0,5 N. On agite encore 2 heures à cette température, extrait à environ 5°C par 3 fois 600 cm3 d'éther éthylique. Les phases organiques réunies sont lavées par 3 fois 500 cm3 d'eau, séchées sur sulfate de magnésium, et concentrées sous pression réduite (20kPa) à une température voisine de 30°C. L'extrait sec est repris par un mélange de 135 cm3 d'éther isopropylique et de 15 cm3 de n-hexane, essoré à environ 5°C, lavé par 2 fois 115 cm3 du même mélange à la même température. On obtient 47,4 g de (chloro-2 trifluoro-6,7,8 quinolyl-3)-3 oxo-3 propionate d'éthyle sous forme d'un solide beige fondant à 78-80°C qui est utilisé sans autre purification pour les étapes ultérieures.

L'acide chloro-2 trifluoro-6,7,8 quinoléine carboxylique-3 est préparé de la manière suivante :

A une suspension, sous agitation, refroidie à environ 10°C, de 45,7 g de chloro-2 trifluoro-6,7,8 formyl-3 dihydro-1,4 quinoléine dans 585 cm3 de potasse N, on ajoute en 1 heure en maintenant la température entre 10 et 14°C, une solution de 69,65 g de permanganate de potassium dans 730 cm3 d'eau. On laisse encore agiter 30 minutes à environ 10°C. On ajoute 12 g de dithionite de sodium, agite 10 minutes à une température voisine de 10°C, filtre sur silice diatomée et lave par 3 fois 400 cm3 d'eau. Le filtrat et les lavages sont réunis et additionnés de 70 cm3 d'acide chlorhydrique en solution aqueuse à 35 %. Le précipité formé est extrait par 3 fois 500 cm3 d'acétate d'éthyle. Les extraits organiques réunis sont séchés sur sulfate de magnésium, filtrés et concentrés sous pression réduite (20kPa) à 50°C. Le résidu est repris par un mélange de 100 cm3 d'éther éthylique et 100 cm3 d'éther isopropylique, essoré, lavé par 100 cm3 du même mélange. On obtient 46,43 g d'acide chloro-2 trifluoro-6,7,8 quinoléine carboxylique-3 sous forme d'un solide incolore se décomposant à 225-230°C qui est utilisé sans autre purification pour les étapes ultérieures.

La chloro-2 trifluoro-6,7,8 formyl-3 dihydro-1,4 quinoléine est préparée de la manière suivante :

A un mélange de 525 cm3 de trichlorométhane et de 49 cm3 de diméthylformamide, on ajoute en 40 minutes, sous agitation, entre 5 et 10°C, 50 cm3 de chlorure de phosphoryle, agite 15 minutes à cette température et laisse la température remonter à environ 20°C. A la solution obtenue, on ajoute en 20 minutes à environ 20°C, progressivement, sous forte agitation, 46,8 g de trifluoro-6,7,8 dihydro-3,4 carbostyrile. On laisse agiter 30 minutes à une température voisine de 20°C, chauffe à environ 60°C et maintient à cette même température pendant 2 heures et demie. Le mélange réactionnel est concentré sous pression réduite (20kPa) à environ 50°C. Le résidu huileux est versé sous forte agitation dans 500 g de glace. On ajoute, par petites fractions, en 30 minutes, 100 g d'acétate de sodium. La suspension obtenue est versée en 15 minutes, sous forte agitation dans 1 litre d'eau préalablement chauffée à environ 90°C et agite encore 15 minutes à cette même température. L'insoluble est essoré à environ 90°C et lavé par 3 fois 250 cm3 d'eau. On obtient 47,7 g de chloro-2 trifluoro-6,7,8 formyl-3 dihydro-1,4 quinoléine sous forme d'un solide incolore se décomposant à 220°C.

Le trifluoro 6,7,8 dihydro-3,4 carbostyrile est préparé de la manière suivante :

24,35 g de trifluoro-6,7,8 carbostyrile en suspension dans un mélange de 450 cm3 d'éthanol et 150 cm3 de diméthylformamide sont hydrogénés, sous agitation, à environ 50°C, en présence de 5 g de nickel de Raney sous une pression de 1 atmosphère, jusqu'à fin d'absorption d'hydrogène. Le nickel de Raney utilisé de qualité W-2 est préalablement lavé par 50 cm3 d'une solution d'acide acétique aqueux à 2 %, par 2 fois 50 cm3 d'eau et par 3 fois 50 cm3 d'éthanol. Le mélange réactionnel est additionné de 250 cm3 de diméthylformamide, filtré

à environ 50°C sur silice diatomée. Le filtrat est concentré sous pression réduite (20kPa) à environ 70°C. L'extrait sec est repris par 150 cm3 d'eau, essoré et lavé par 2 fois 50 cm3 d'eau. On obtient 23,6 g de trifluoro-6,7,8 dihydro-3,4 carbostyrile sous forme d'un solide beige clair fondant à 217°C qui est utilisé sans autre purification pour les étapes ultérieures.

Le trifluoro-6,7,8 carbostyrile est préparé de la manière suivante :

60,83 g de chloro-4 trifluoro-6,7,8 carbostyrile en suspension dans 520 cm3 d'acide acétique et 38,15 cm3 de triéthylamine sont hydrogénés sous une pression de 1 atmosphère, en présence de 5,25 g de palladium sur charbon à 10 % jusqu'à fin d'absorption d'hydrogène, à une température voisine de 25°C. Le mélange réactionnel est ensuite chauffé à environ 40°C, filtré à cette même température, sur silice diatomée pour filtration. Le filtrat est concentré sous pression réduite (20kPa) à une température voisine de 50°C. L'extrait sec est repris par 400 cm3 d'eau ; l'insoluble est essoré, lavé par 4 fois 170 cm3 d'eau, 2 fois 110 cm3 d'éthanol et 2 fois par 100 cm3 d'éther isopropylique. On obtient 48,35 g de trifluoro-6,7,8 carbostyrile, sous forme d'un solide incolore se sublimant à 288°C, qui est utilisé sans autre purification pour les étapes ultérieures.

Le chloro-4 trifluoro-6,7,8 carbostyrile est préparé de la manière suivante :

Une suspension de 70,4 g de chloro-4 éthoxy-2 trifluoro-6,7,8 quinoléine dans 170 cm3 d'une solution aqueuse d'acide chlorhydrique à 35 %, 420 cm3 d'acide acétique et 250 cm3 d'eau est chauffée sous agitation à une température voisine de 100°C pendant 2 heures et demie. Après refroidissement à environ 20°C, le mélange réactionnel est versé dans 1100 cm3 d'eau à environ 5°C, agité 15 minutes à cette même température, puis l'insoluble est essoré et lavé par 3 fois 220 cm3 d'eau. On obtient 61 g de chloro-4 trifluoro-6,7,8 carbostyrile, sous forme d'un solide crème fondant à 213°C qui est utilisé sans autre purification pour les étapes ultérieures.

La chloro-4 éthoxy-2 trifluoro-6,7,8 quinoléine est préparée de la manière suivante :

Une suspension de 69,5 g d'éthoxy-2 trifluoro-6,7,8 hydroxy-4 quinoléine dans 430 cm3 de chlorure de phosphoryle est chauffée, sous agitation à une température voisine de 100°C pendant 30 minutes. La solution obtenue est concentrée, sous pression réduite (20kPa) à environ 60°C, jusqu'à un volume de 100 cm3. Le résidu est repris par 750 cm3 d'acétate d'éthyle ; la solution obtenue est versée sous agitation, en 10 minutes, dans un mélange de 400 cm3 d'eau et 200 g de glace et laisse agiter dans ces conditions pendant 30 minutes. Après séparation de l'extrait organique, la phase aqueuse est de nouveau extraite par 2 fois 250 cm3 d'acétate d'éthyle. Les extraits organiques réunis sont lavés par 3 fois 250 cm3 d'eau, séchés sur sulfate de magnésium et concentrés sous pression réduite (20kPa) à environ 40°C. Le résidu huileux obtenu est repris par 370 cm3 d'éther de pétrole ((40-60). Après filtration sur silice diatomée, le filtrat est concentré à sec, sous pression réduite (20kPa) à environ 30°C. On obtient 70,7 g de chloro-4 éthoxy-2 trifluoro-6,7,8 quinoléine, sous forme d'un solide beige fondant à 45°C qui est utilisé sans autre purification pour les étapes ultérieures.

L'éthoxy-2 trifluoro-6,7,8 hydroxy-4 quinoléine peut être préparée de la manière suivante :

Une solution de 122 g de trifluoro-2,3,4 N-[(éthoxy-1′ éthoxycarbonyl-2′) éthylidène]-aniline dans 120 cm3 d'oxyde de phényle est introduite, goutte à goutte, en 25 minutes, sous agitation, dans 600 cm3 d'oxyde de phényle à une température voisine de 250°C tout en éliminant l'éthanol formé par distillation. Après agitation pendant 15 minutes à cette même température, la solution est refroidie à environ 20°C et additionnée de 750 cm3 de n-hexane. Le précipité formé est essoré et lavé 3 fois par 200 cm3 de n-hexane. On obtient 69,5 g d'éthoxy-2 trifluoro-6,7,8 hydroxy-4 quinoléine sous forme d'un solide beige fondant à 171°C qui est utilisé sans autre purification pour les étapes ultérieures.

La trifluoro-2,3,4 N-[(éthoxy-1′ éthoxycarbonyl-2′) éthylidène]-aniline peut être préparée de la manière suivante :

A une solution de 90 g de chlorhydrate d'(éthoxycarbonyl-2 éthoxy-1) éthylidèneamine dans 820 cm3 d'éthanol, on ajoute en une seule fois, sous agitation, 58,8 g de trifluoro-2,3,4 aniline. Après 48 heures d'agitation à une température voisine de 20°C, la suspension obtenue est filtrée ; le filtrat est concentré sous pression réduite (20kPa) à une température voisine de 50°C. Le résidu huileux est repris par 250 cm3 d'eau. Le mélange obtenu est extrait par 3 fois 200 cm3 d'éther éthylique. Les extraits organiques réunis sont lavés par 4 fois 150 cm3 d'eau, séchés sur sulfate de magnésium et concentrés sous pression réduite (20kPa) à environ 30°C. On obtient 122 g de trifluoro-2,3,4 N-[(éthoxy-1′ éthoxycarbonyl-2′)éthylidène]-aniline sous forme d'une huile jaune qui est utilisée sans autre purification pour les étapes ultérieures.

Le chlorhydrate d'(éthoxycarbonyl-2 éthoxy-1) éthylidèneamine a été préparé selon la méthode décrite par A. Pinner, et coll., Ber. Dtsch. Chem. Ges., 28, 478 (1895).

EXEMPLE 10

L'acide trifluoro-6,7,8 méthoxy-1 oxo-4 dihydro-1,4 benzo[b] naphtyridine-1,8 carboxylique-3 a été préparé dans les conditions de l'exemple 9, mais à partir de 9 g d'éthoxycarbonyl-3 trifluoro-6,7,8 méthoxy-1 oxo-4 dihydro-1,4 benzo[b]naphtyridine-1,8. On obtient 7,7 g d'acide trifluoro-6,7,8 méthoxy-1 oxo-4 dihydro-1,4 benzo[b]naphtyridine-1,8 carboxylique-3 sous forme d'un solide beige fondant à 322°C.

L'éthoxycarbonyl-3 trifluoro-6,7,8 méthoxy-1 oxo-4 dihydro-1,4 benzo [b] naphtyridine-1,8 a été préparé dans les conditions suivantes:

Une suspension de 5,1 g de chlorhydrate de méthylhydroxylamine dans 120 cm3 de trichlorométhane est additionnée de 8,7 cm3 de triéthylamine. A la solution obtenue, on ajoute à environ 20°C, 7,8 g de (chloro-2 trifluoro-6,7,8 quinoléinecarbonyle-3)-2 diméthylamino-3 acrylate d'éthyle. Après 2 heures d'agitation à cette température, la solution est concentrée à sec, sous pression réduite (20kPa) à environ 50°C. Le résidu obtenu est repris par 150 cm3 d'éthanol et 10 cm3 de triéthylamine et chauffé, sous agitation, pendant 30 minutes. Après refroidissement à environ 20°C, l'insoluble est essoré, lavé par 3 fois 50 cm3 d'éthanol et 2 fois 50 cm3 d'éther isopropylique. Après 1 recristallisation dans 120 cm3 de diméthylformamide, on obtient 9 g d'éthoxycarbonyl-3 trifluoro-6,7,8 méthoxy-1 oxo-4 dihydro-1,4 benzo[b]naphtyridine-1,8 sous forme d'un solide jaune fondant à 298-300°C.

## EXEMPLE 11

En opérant dans les conditions de l'exemple 9, mais à partir de 1,8 g de cyclopropyl-1 éthoxycarbonyl-3 trifluoro-7,8,9 oxo-4 dihydro-1,4 benzo[b]naphtyridine-1,8. On obtient 1,1 g d'acide cyclopropyl-1 trifluoro-7,8,9 oxo-4 dihydro-1,4 benzo[b]naphtyridine-1,8 carboxylique-3 sous forme d'un solide jaune fondant à 304°C.

La cyclopropyl-1 éthoxycarbonyl-3 trifluoro-7,8,9 oxo-4 dihydro-1,4 benzo[b]naphtyridine-1,8 peut être préparée de la manière suivante :

Dans une solution de 7 g de (chloro-2 trifluoro-6,7,8 quinoléinecarbonyle-3)-2 diméthylamino-3 acrylate d'éthyle dans 100 cm3 de trichlorométhane maintenue à une température voisine de 20°C, on ajoute en 5 minutes 4,12 g de cyclopropylamine et agite encore 4 heures à cette même température. Le mélange réactionnel est concentré sous pression réduite (20kPa) à environ 50°C. Le résidu huileux obtenu est repris par 100 cm3 d'éthanol et 3 g de D.B.U. Le mélange est chauffé à 80°C et maintenu, sous agitation, à cette même température pendant 1 heure et demie. Après refroidissement à environ 20°C l'insoluble est essoré, lavé par 2 fois 30 cm3 d'éthanol et 2 fois 30 cm3 d'éther isopropylique. On obtient 4,5 g de cyclopropyl-1 éthoxycarbonyl-3 trifluoro-7,8,9 oxo-4 dihydro-1,4 benzo [b] naphtyridine-1,8 sous forme d'un solide incolore fondant à 260°C.

## EXEMPLE 12

Une suspension de 6 g de chloro-8 éthoxycarbonyl-3 éthoxyméthyl-1 fluoro-7 oxo-4 dihydro-1,4 benzo[b]naphtyridine-1,8 dans 120 $cm^3$ d'eau, 120 $cm^3$ d'éthanol et 47,5 $cm^3$ de potasse aqueuse 2N est chauffée, sous agitation, à une température voisine de 75°C pendant 2 heures et demie. Un léger insoluble est éliminé par filtration à cette même température. Après refroidissement à environ 20°C, le filtrat est additionné de 6 $cm^3$ d'acide acétique. Le précipité obtenu et essoré, lavé par 3 fois 20 $cm^3$ d'eau et recristallisé dans 60 $cm^3$ de diméthylformamide. On obtient 4 g d'acide chloro-8 éthoxyméthyl-1 fluoro-7 oxo-4 dihydro-1,4 benzo[b]naphtyridine-1,8 carboxylique-3 sous forme d'un solide jaune se décomposant à 285°C.

La chloro-8 éthoxycarbonyl-3 éthoxyméthyl-1 fluoro-7 oxo-4 dihydro-1,4 benzo[b]naphtyridine-1,8 est préparée de la manière suivante :

Une suspension de 6 g de chloro-8 éthoxycarbonyl-3 fluoro-7 oxo-4 dihydro-1,4 benzo[b]naphtyridine-1,8 et de 5,16 g de carbonate de potassium dans 120 $cm^3$ de diméthylformamide est chauffée à une température voisine de 110°C pendant 1 heure. Après refroidissement à environ 15°C, on ajoute 5,44 $cm^3$ de chlorométhyl éthyl éther et agite 5 heures entre 15 et 20°C. Le mélange réactionnel est concentré à sec, sous pression réduite (20 kPa) à environ 60°C. Le résidu est repris par 3 fois 150 $cm^3$ de trichlorométhane. Les extraits organiques réunis sont filtrés afin d'éliminer l'insoluble. Le filtrat est séché sur sulfate de magnésium et concentré sous pression réduite (20 kPa) à environ 40°C. Le résidu est recristallisé dans 55 $cm^3$ de diméthylformamide. On obtient 5,6 g de chloro-8 éthoxycarbonyl-3 éthoxyméthyl-1 fluoro-7 oxo-4 dihydro-1,4 benzo[b]naphtyridine-1,8 sous forme d'un solide beige fondant à 261°C.

La chloro-8 éthoxycarbonyl-3 fluoro-7 oxo-4 dihydro-1,4 benzo[b]naphtyridine-1,8 a été préparée de la manière suivante :

Un mélange de 11,3 g de (dichloro-2,7 fluoro-6 quinoléinecarbonyle-3)-2 diméthylamino-3 acrylate d'éthyle et 5,65 g d'amino-3 triazine-1,2,4 dans 60 $cm^3$ de trichlorométhane est agité 16 heures à une température voisine de 20°C. Le mélange réactionnel est concentré à sec, sous pression réduite (20°C) à une température voisine de 30°C. Le résidu est repris par 60 $cm^3$ d'éthanol et 5,6 g de DBU et chauffé à une température voisine de 75°C pendant 20 heures. Après refroidissement à 20°C, l'insoluble est essoré et lavé par 2 fois 40 $cm^3$

d'éthanol. On obtient 6,1 g de chloro-8 éthoxycarbonyl-3 fluoro-7 oxo-4 dihydro-1,4 benzo[b]naphtyridine-1,8 sous forme d'un solide brun se décomposant à 378-380°C qui est utilisé sans autre purification pour les étapes ultérieures.

EXEMPLE 13

Une suspension de 5,75 g de chloro-8 éthoxycarbonyl-3 fluoro-7 oxo-4 dihydro-1,4 benzo[b]naphtyridine-1,8 dans 60 $cm^3$ d'acide chlorhydrique à 17,5 % est chauffée, sous agitation, à une température voisine de 100°C pendant 30 minutes. Après refroidissement à environ 20°C, l'insoluble est essoré, lavé par 3 fois 20 $cm^3$ d'éthanol et 3 fois 20 $cm^3$ d'éther éthylique. On obtient 3,05 g d'acide chloro-8 fluoro-7 oxo-4 dihydro-1,4 benzo[b]naphtyridine-1,8 carboxylique-3 sous forme d'un solide beige se décomposant à 416°C.

EXEMPLE 14

Une suspension de 8 g d'éthoxycarbonyl-3 éthyl-1 difluoro-7,8 oxo-4 dihydro-1,4 benzo[b]naphtyridine-1,8 dans 80 $cm^3$ d'acide chlorhydrique à 17,5 % et 80 $cm^3$ d'acide acétique est chauffée, sous agitation, à une température voisine de 100°C pendant 1 heure et 30 minutes. Après refroidissement à environ 20°C, l'insoluble est essoré, lavé par 3 fois 20 $cm^3$ d'eau et recristallisé dans 50 $cm^3$ de diméthylformamide. On obtient 6,3 g d'acide éthyl-1 difluoro-7,8 oxo-4 dihydro-1,4 benzo[b]naphtyridine-1,8 carboxylique-3 sous forme d'un solide jaune fondant à 330°C.

L'éthoxycarbonyl-3 éthyl-1 difluoro-7,8 oxo-4 dihydro-1,4 benzo[b]naphtyridine-1,8 a été préparé dans les conditions suivantes :

Dans une suspension de 20 g de (chloro-2 difluoro-6,7 quinoléinecarbonyle-3)-2 diméthylamino-3 acrylate d'éthyle dans 200 cm3 d'éthanol à environ 2°C, on ajoute, en 10 minutes, entre 2 et 5°C, sous agitation, une solution à environ 2°C de 14,6 g d'éthylamine dans 200 cm3 d'éthanol, agite encore 40 minutes entre 2 et 5°C puis laisse la température remonter à environ 20°C en 2 heures. Après 24 heures à environ 20°C, l'insoluble est essoré, lavé par 2 fois 30 cm3 d'éthanol et 2 fois 50 cm3 d'éther isopropylique. On obtient 16,35 g d'éthoxycarbonyl-3 éthyl-1 difluoro-7,8 oxo-4 dihydro-1,4 benzo [b] naphtyridine-1,8 sous forme d'un solide beige fondant à 290°C.

EXEMPLE 15

L'acide chloro-8 fluoro-7 (fluoro-2 éthyl)-1 oxo-4 dihydro-1,4 benzo [b] naphtyridine-1,8 carboxylique-3 est préparé dans les conditions de l'exemple 9, mais à partir de 2,2 g de chloro-8 éthoxycarbonyl-3 fluoro-7 (fluoro-2 éthyl)-1 oxo-4 dihydro-1,4 benzo [b] naphtyridine-1,8. Après 2 recristallisations dans 10 cm3 de diméthylformamide à chaque fois, on obtient 1,4 g d'acide chloro-8 fluoro-7 (fluoro-2 éthyl)-1 oxo-4 dihydro-1,4 benzo[b]naphtyridine-1,8 carboxylique-3 sous forme d'un solide jaune fondant à 310°C.

La chloro-8 éthoxycarbonyl-3 fluoro-7 (fluoro-2 éthyl)-1 oxo-4 dihydro-1,4 benzo [b] naphtyridine-1,8 est préparée de la manière suivante:

Une suspension de 1,9 g de chlorhydrate de fluoro-2 éthylamine dans 25 cm3 de trichlorométhane est additionnée de 2,7 cm3 de triéthylamine. A la solution obtenue, on ajoute à environ 20°C, 3,5 g de (dichloro-2,7 fluoro-6 quinoléinecarbonyle-3)-2 diméthylamino-3 acrylate d'éthyle. Après 16 heures d'agitation à cette température, la solution est concentrée à sec sous pression réduite (20kPa) à environ 50°C. Le résidu est repris par 20 cm3 d'éthanol et 3 cm3 de triéthylamine et chauffé à environ 75°C, sous agitation pendant 2 heures. Après refroidissement à environ 20°C, l'insoluble est essoré, lavé par 2 fois 10 cm3 d'éthanol, 2 fois 10 cm3 d'éther isopropylique. On obtient 1,9 g de chloro-8 éthoxycarbonyl-3 fluoro-7 (fluoro-2 éthyl)-1 oxo-4 dihydro-1,4 benzo [b]naphtyridine-1,8 sous forme d'un solide jaune fondant à 268°C qui est utilisé sans autre purification pour l'étape ultérieure.

Les produits selon l'invention peuvent être utilisés dans les conditions décrites ci-après à titre d'exemple :

EXEMPLE DE REFERENCE 1

Une suspension de 3,5 g d'acide chloro-8 fluoro-7 méthyl-1 oxo-4 dihydro-1,4 benzo[b]naphtyridine-1,8 carboxylique-3 et de 4,6 g de méthyl-2 pipérazine dans 40 cm3 de pyridine est chauffée à température voisine de 115°C sous agitation pendant 13 heures. Le mélange réactionnel est concentré à sec sous pression réduite (20 kPa) à 60°C. Le résidu est repris 2 fois par 30 cm3 d'éthanol et concentré sous pression réduite dans les conditions ci-dessus. Le solide obtenu est repris par 60 cm3 d'eau et 10 cm3 de potasse aqueuse à 30 %. La phase aqueuse est lavée par 2 fois 100 cm3 de trichlorométhane, additionnée de 10,28 g d'acide méthanesulfonique lavée de nouveau par 2 fois 100 cm3 de trichlorométhane. On ajoute 10 cm3 de potasse aqueuse à 30 %. Le précipité formé est essoré, lavé par 3 fois 10 cm3 d'eau et 2 fois 10 cm3 d'éthanol. On obtient 2,7 g d'acide fluoro-7 méthyl-1 (méthyl-3 pipérazinyl-1)-8 oxo-4 dihydro-1,4 benzo[b]naphtyridine-1,8 carboxylique-3 sous forme de solide jaune fondant à 360-363°C.

EXEMPLE DE REFERENCE 2

L'acide fluoro-7 méthyl-1 oxo-4 (pipérazinyl-1)-8 dihydro-1,4 benzo[b]naphtyridine-1,8 carboxylique-3 est préparé dans les conditions de l'exemple de référence 1 mais à partir de 10 g d'acide chloro-8 fluoro-7 méthyl-1 oxo-4 dihydro-1,4 benzo[b]naphtyridine-1,8 carboxylique-3 et de 28 g de pipérazine, dans 100 cm3 de pyridine. On obtient 5,5 g d'hémihydrate de l'acide fluoro-7 méthyl-1 oxo-4 (pipérazinyl-1)-8 dihydro-1,4 benzo[b]naphtyridine-1,8 carboxylique-3 sous forme d'un solide jaune fondant à 370-375°C.

EXEMPLE DE REFERENCE 3

L'acide fluoro-7 méthyl-1 (méthyl-4 pipérazinyl-1)-8 oxo-4 dihydro-1,4 benzo[b]naphtyridine-1,8 carboxylique-3 est préparé dans des conditions analogues à l'exemple de référence 1 mais à partir de 5 g d'acide chloro-8 fluoro-7 méthyl-1 oxo-4 dihydro-1,4 benzo[b]naphtyridine-1,8 carboxylique-3 et de 16 g de méthyl-1 pipérazine dans 50 cm3 de pyridine. Après concentration du mélange réactionnel sous pression réduite le résidu mis en suspension dans 100 cm3 d'eau est additionné de 25 cm3 d'acide acétique. On élimine un très léger insoluble par filtration sur silice diatomée pour filtration. On ajoute au filtrat 200 cm3 de potasse aqueuse 3N, élimine de nouveau un très léger insoluble par filtration sur silice diatomée pour filtration. On ajoute au filtrat 5 cm3 d'acide acétique. Le précipité formé est essoré, lavé par 3 fois 50 cm3 d'eau. Après 2 recristallisations dans 17 cm3 de diméthylformamide à chaque fois, on obtient 3,2 g d'acide fluoro-7 méthyl-1 (méthyl-4 pipérazinyl-1)-8 oxo-4 dihydro-1,4 benzo[b]naphtyridine-1,8 carboxylique-3 sous forme d'un solide jaune fondant à 356°C.

EXEMPLE DE REFERENCE 4

L'acide (éthyl-4 pipérazinyl-1)-8 fluoro-7 méthyl-1 oxo-4 dihydro-1,4 benzo[b]naphtyridine-1,8 carboxylique-3 est préparé dans les conditions décrites ci-après à l'exemple de référence 5, mais à partir de 1,85 g d'acide chloro-8 fluoro-7 méthyl-1 oxo-4 dihydro-1,4 benzo[b]naphtyridine-1,8 carboxylique-3 et de 2,75 g d'éthyl-1 pipérazine, dans 20 cm3 de pyridine. On obtient 1,3 g d'acide (éthyl-4 pipérazinyl-1)-8 fluoro-7 méthyl-1 oxo-4 dihydro-1,4 benzo[b]naphtyridine-1,8 carboxylique-3 sous forme d'un solide jaune fondant à 285-286°C.

EXEMPLE DE REFERENCE 5

L'acide fluoro-7 [(hydroxy-2 éthyl)-4 pipérazinyl-1]-8 méthyl-1 oxo-4 dihydro-1,4 benzo[b]naphtyridine-1,8 carboxylique-3 est préparé dans les conditions de l'exemple de référence 1 mais à partir de 1,6 g d'acide chloro-8 fluoro-7 méthyl-1 oxo-4 dihydro-1,4 benzo[b]naphtyridine-1,8 carboxylique-3 et de 6,8 g d'(hydroxy-2 éthyl)-1 pipérazine dans 16 cm3 de pyridine. Après concentration à sec du mélange réactionnel sous pression réduite, le résidu est repris par 50 cm3 d'eau. Le mélange est amené à pH 6,9 par addition de 0,4 cm3 d'acide acétique. Le précipité obtenu est essoré, lavé par 2 fois 10 cm3 d'eau, recristallisé 2 fois dans 10 cm3 de diméthylformamide. On obtient 1,1 g d'acide fluoro-7 [(hydroxy-2 éthyl)-4 pipérazinyl-1]-8 méthyl-1 oxo-4 dihydro-1,4 benzo[b]naphtyridine-1,8 carboxylique-3 sous forme d'un solide jaune fondant à 275-276°C.

EXEMPLE DE REFERENCE 6

L'acide (diméthyl-3,5 pipérazinyl-1)-8 fluoro-7 méthyl-1 oxo-4 dihydro-1,4 benzo[b]naphtyridine-1,8 carboxylique-3 est préparé dans les conditions de l'exemple de référence 3 mais à partir de 1,7 g d'acide chloro-8 fluoro-7 méthyl-1 oxo-4 dihydro-1,4 benzo[b]- naphtyridine-1,8 carboxylique-3 et de 2,5 g de diméthyl-2,6 pipérazine, dans 20 cm3 de pyridine. On obtient 1,1 g d'hémihydrate de l'acide (diméthyl-3,5 pipérazinyl-1)-8 fluoro-7 méthyl-1 oxo-4 dihydro-1,4 benzo[b]naphtyridine-1,8 carboxylique-3 sous forme d'un solide jaune fondant à 294-295°C.

EXEMPLE DE REFERENCE 7

L'acide éthyl-1 fluoro-7 (pipérazinyl-1)-8 oxo-4 dihydro-1,4 benzo[b]naphtyridine-1,8 carboxylique-3 est préparé dans les conditions de l'exemple de référence 5 mais à partir de 1,6 g d'acide chloro-8 éthyl-1 fluoro-7 oxo-4 dihydro-1,4 benzo[b]naphtyridine-1,8 carboxylique-3 et de 4,3 g de pipérazine dans 20 cm3 de pyridine. Après 3 recristallisations dans, au total, 300 cm3 de diméthylformamide, on obtient 0,94 g de trihydrate de l'acide éthyl-1 fluoro-7 (pipérazinyl-1)-8 oxo-4 dihydro-1,4 benzo[b]naphtyridine-1,8 carboxylique-3 sous forme d'un solide jaune fondant à 320-322°C.

EXEMPLE DE REFERENCE 8

L'acide éthyl-1 fluoro-7 (méthyl-4 pipérazinyl-1)-8 oxo-4 dihydro-1,4 benzo[b]naphtyridine-1,8 carboxylique-3 est préparé dans les conditions de l'exemple de référence 5 mais à partir de 1,6 g d'acide chloro-8 éthyl-1 fluoro-7 oxo-4 dihydro-1,4 benzo[b]naphtyridine-1,8 carboxylique-3 et de 4,5 g de méthyl-4 pipérazine dans 16 cm3 de pyridine. Après 4 recristallisations dans, au total, 120 cm3 de diméthylformamide, on obtient 1,2 g d'acide éthyl-1 fluoro-7 (méthyl-4 pipérazinyl-1)-8 oxo-4 dihydro-1,4 benzo[b]- naphtyridine-1,8 carboxylique-3 sous forme d'un solide jaune fondant à 285-286°C solvaté par 1 % d'eau.

EXEMPLE DE REFERENCE 9

L'acide éthyl-1 fluoro-7 (méthyl-3 pipérazinyl-1)-8 oxo-4 dihydro-1,4 benzo[b]naphtyridine-1,8 carboxylique-3 est préparé dans les conditions de l'exemple de référence 1 mais à partir de 2,1 g d'acide chloro-8 éthyl-1 fluoro-7 oxo-4 dihydro-1,4 benzo[b]naphtyridine-1,8 carboxylique-3, de 20 cm3 de pyridine, et de 2,4 g de méthyl-2 pipérazine.

Après reprise par l'éthanol et concentration à sec sous pression réduite (20 kPa à 50°C), le résidu solide est repris par 20 cm3 d'eau et 10 cm3 de potasse 2N. La solution aqueuse obtenue est lavée par 2 fois 20 cm3 de trichlorométhane, additionnée de 10 cm3 d'acide acétique, lavée de nouveau par 2 fois 40 cm3 de trichlorométhane. On ajoute 23 cm3 de potasse 4,5 N, chauffe la suspension obtenue à une température voisine de 90°C. Après refroidissement à une température voisine de 20°C, le précipité est essoré, lavé par 3 fois 10 cm3 d'eau, 2 fois 10 cm3 d'éthanol. Après 2 recristallisations dans 120 cm3 de diméthylformamide à chaque fois, on obtient 1,7 g d'acide éthyl-1 fluoro-7 (méthyl-3 pipérazinyl-1)-8 oxo-4 dihydro-1,4 benzo[b]naphtyridine-1,8 carboxylique-3 sous forme d'un solide jaune fondant à 310-312°C.

EXEMPLE DE REFERENCE 10

L'acide éthyl-1 (éthyl-4 pipérazinyl-1)-8 fluoro-7 oxo-4 dihydro-1,4 benzo[b]naphtyridine-1,8 carboxylique-3 est préparé dans les conditions de l'exemple de référence 5, mais à partir de 1,6 g d'acide chloro-8 éthyl-1 fluoro-7 oxo-4 dihydro-1,4 benzo[b]naphtyridine-1,8 carboxylique-3 et de 2,3 g d'éthyl-1 pipérazine dans 16 cm3 de pyridine. On obtient 1,4 g d'acide éthyl-1 (éthyl-4-pipérazinyl-1)-8 fluoro-7 oxo-4 dihydro-1,4 benzo[b]naphtyridine-1,8 carboxylique-3 sous forme d'un solide jaune fondant à 287-288°C, solvaté par 1,6 % d'eau.

EXEMPLE DE REFERENCE 11

L'acide éthyl-1 fluoro-7 [(hydroxy-2 éthyl)-4 pipérazinyl-1]-8 oxo-4 dihydro-1,4 benzo[b]naphtyridine-1,8 carboxylique-3 est préparé dans les conditions de l'exemple de référence 5, mais à partir de 1,6 g de l'acide chloro-8 éthyl-1 fluoro-7 oxo-4 dihydro-1,4 benzo[b]naphtyridine-1,8 carboxylique-3 et de 2,6 g de (hydroxy-2 éthyl)-1 pipérazine dans 16 cm3 de pyridine. On obtient 1,3 g d'acide éthyl-1 fluoro-7 [(hydroxy-2 éthyl)-4 pipérazinyl-1]-8 oxo-4 dihydro-1,4 benzo[b]naphtyridine-1,8 carboxylique-3 sous forme d'un solide jaune fondant à 264-265°C.

EXEMPLE DE REFERENCE 12

L'acide fluoro-7 méthylamino-1 oxo-4 (pipérazinyl-1)-8 dihydro-1,4 benzo[b]naphtyridine-1,8 carboxylique-3 est préparé dans les conditions de l'exemple de référence 5 mais à partir de 2,25 g d'acide chloro-8 fluoro-7 méthylamino-1 oxo-4 dihydro-1,4 benzo[b]naphtyridine-1,8 carboxylique-3 et de 2,4 g de pipérazine dans 30 cm3 de pyridine. Après 3 recristallisations dans, au total, 400 cm3 de diméthylformamide, on obtient 0,82 g d'acide fluoro-7 méthylamino-1 oxo-4 (pipérazinyl-1)-8 dihydro-1,4 benzo[b]naphtyridine-1,8 carboxylique-3 sous forme d'un solide jaune foncé fondant à 322-324°C solvaté par 13,6 % de diméthylformamide.

EXEMPLE DE REFERENCE 13

L'acide fluoro-7 méthylamino-1 (méthyl-4 pipérazinyl-1)-8 oxo-4 dihydro-1,4 benzo[b]naphtyridine-1,8 carboxylique-3 est préparé dans des conditions analogues à l'exemple de référence 1 mais à partir de 1,93 g d'acide chloro-8 fluoro-7 méthylamino-1 oxo-4 dihydro-1,4 benzo[b]naphtyridine-1,8 carboxylique-3, de 2,4 g de méthyl-1 pipérazine, de 20 cm3 de pyridine. Après 2 recristallisations dans 15 cm3 de diméthylformamide à chaque fois, on obtient 0,9 g d'acide fluoro-7 méthylamino-1 (méthyl-4 pipérazinyl-1)-8 oxo-4 dihydro-1,4 benzo[b]naphtyridine-1,8 carboxylique-3 sous forme d'un solide jaune fondant à 263-264°C.

EXEMPLE DE REFERENCE 14

L'acide fluoro-7 méthylamino-1 (méthyl-3 pipérazinyl-1)-8 oxo-4 dihydro-1,4 benzo[b]naphtyridine-1,8 carboxylique-3 est préparé dans les conditions de l'exemple de référence 5 mais à partir de 3,2 g d'acide chloro-8 fluoro-7 méthylamino-1 oxo-4 dihydro-1,4 benzo[b]naphtyridine-1,8 carboxylique-3 et de 4 g de méthyl-2 pipérazine dans 40 cm3 de pyridine. Le produit brut obtenu est repris par 30 cm3 d'eau et 7 cm3 de potasse aqueuse 2N. On élimine un très léger insoluble par filtration sur silice diatomée. Le filtrat est lavé par 2 fois 20 cm3 d'éther éthylique puis précipité par addition de 3,5 cm3 d'acide méthanesulfonique 4N. Le précipité obtenu est essoré, lavé par 3 fois 20 cm3 d'eau,3 fois 20 cm3 d'éthanol. On obtient 2,2 g d'acide fluoro-7 méthylamino-1 (méthyl-3 pipérazinyl-1)-8 oxo-4 dihydro-1,4 benzo[b]naphtyridine-1,8 carboxylique-3 sous forme d'un solide jaune foncé fondant à 343-345°C, solvaté par 3,7 % d'eau.

EXEMPLE DE REFERENCE 15

L'acide cyclopropyl-1 fluoro-7 oxo-4 (pipérazinyl-1)-8 dihydro-1,4 benzo[b]naphtyridine-1,8 carboxylique-3 est préparé dans les conditions de l'exemple de référence 5, mais à partir de 1 g d'acide chloro-8 cyclopropyl-1 fluoro-7 oxo-4 dihydro-1,4 benzo[b]-naphtyridine-1,8 carboxylique-3 et de 2,6 g de pipérazine, dans 10 cm3 de pyridine. On obtient 0,6 g de dihydrate de l'acide cyclo- propyl-1 fluoro-7 oxo-4 (pipérazinyl-1)-8 dihydro-1,4 benzo[b]naphtyridine-1,8 carboxylique-3 sous forme d'un solide jaune fondant à 342-343°C.

EXEMPLE DE REFERENCE 16

L'acide cyclopropyl-1 fluoro-7 (méthyl-4 pipérazinyl-1)-8 oxo-4 dihydro-1,4 benzo[b]naphtyridine-1,8 carboxylique-3 est préparé dans les conditions de l'exemple de référence 5 mais à partir de 1 g d'acide chloro-8 cyclopropyl-1 fluoro-7 oxo-4 dihydro-1,4 benzo[b]- naphtyridine-1,8 carboxylique-3, de 3 g de méthyl-1 pipérazine dans 10 cm3 de pyridine. Après une recristallisation dans 10 cm3 de diméthylformamide, on obtient 0,63 g d'acide cyclopropyl-1 fluoro-7 (méthyl-4 pipérazinyl-1)-8 oxo-4 dihydro-1,4 benzo[b]naphtyridine-1,8 carboxylique-3 sous forme d'un solide jaune fondant à 250°C.

EXEMPLE DE REFERENCE 17

L'acide cyclopropyl-1 fluoro-7 (méthyl-3 pipérazinyl-1)-8 oxo-4 dihydro-1,4 benzo [b] naphtyridine-1,8 carboxylique-3 est préparé dans les conditions de l'exemple de référence 1 mais à partir de 1 g d'acide chloro-8 cyclopropyl-1 fluoro-7 oxo-4 dihydro-1,4 benzo[b]naphtyridine-1,8 carboxylique-3, de 3 g de méthyl-2 pipérazine, dans 10 cm3 de pyridine. Le produit pur est obtenu après une purification supplémentaire par recristallisation dans 200 cm3 de diméthylformamide. On obtient 0,5 g d'hémihydrate de l'acide cyclopropyl-1 fluoro-7 (méthyl-3 pipérazinyl-1)-8 oxo-4 dihydro-1,4 benzo- [b]naphtyridine-1,8 carboxylique-3 sous forme d'un solide jaune fondant à 343°C.

EXEMPLE DE REFERENCE 18

L'acide cyclopropyl-1 (éthyl-4 pipérazinyl-1)-8 fluoro-7 oxo-4 dihydro-1,4 benzo[b]naphtyridine-1,8 carboxylique-3 est préparé dans les conditions de l'exemple de référence 5, mais à partir de 2 g d'acide chloro-8 cyclopropyl-1 fluoro-7 oxo-4 dihydro-1,4 benzo[b]- naphtyridine-1,8 carboxylique-3 et de 2,74 g d'éthyl-1 pipérazine dans 20 cm3 de pyridine. Le produit pur est isolé après une première recristallisation dans 105 cm3 d'éthanol à 25 % de diméthylformamide suivie d'une seconde recristallisation dans 75 cm3 d'éthanol à 50 % de diméthylformamide. On obtient 0,67 g d'acide cyclopropyl-1 (éthyl-4 pipérazinyl-1)-8 fluoro-7 oxo-4 dihydro-1,4 benzo[b]naphtyridine-1,8 carboxylique-3 sous forme d'un solide jaune vert fondant à 254°C.

EXEMPLE DE REFERENCE 19

L'acide cyclopropyl-1 fluoro-7 [(hydroxy-2 éthyl)-4 pipérazinyl-1]-8 oxo-4 dihydro-1,4 benzo[b]naphtyridine-1,8 carboxylique-3 est préparé dans des conditions analogues à l'exemple de référence 5, mais à partir de 4 g d'acide chloro-8 cyclopropyl-1 fluoro-7 oxo-4 dihydro-1,4 benzo[b]naphtyridine-1,8 carboxylique-3 et de 6,2 g d'(hydroxy-2 ethyl)-1 pipérazine dans 40 cm3 de pyridine. Le mélange réactionnel est chauffé pendant 22 heures à une température voisine de 115°C. Le produit pur est isolé après 3 recristallisations dans 3 fois 200 cm3 d'éthanol à 10% de diméthylformamide à chaque fois. On obtient 0,94 g d'acide cyclopropyl-1 fluoro-7 [(hydroxy-2 éthyl)-4 pipérazinyl-1]-8 oxo-4 dihydro-1,4 benzo[b]naphtyridine-1,8 carboxylique-3 sous forme d'un solide jaune fondant à 255°C.

EXEMPLE DE REFERENCE 20

L'acide fluoro-7 oxo-4 (pipérazinyl-1)-8 tertiobutyl-1 dihydro-1,4 benzo[b]naphtyridine-1,8 carboxylique-3 est préparé dans les conditions de l'exemple de référence 5, mais à partir de 1,7 g d'acide chloro-8 fluoro-7 oxo-4 tertiobutyl-1 dihydro-1,4 benzo[b]naphtyridine-1,8 carboxylique-3 et de 4,3 g de pipérazine, dans 20 cm3 de pyridine.Le produit pur est obtenu après une seule recristallisation dans 20 cm3 de diméthylformamide. On isole 1,25 g d'acide fluoro-7 oxo-4 (pipérazinyl-1)-8 tertiobutyl-1 dihydro-1,4 benzo[b]naphtyridine-1,8 carboxylique-3 sous forme d'un solide jaune fondant à 290°C, solvaté par 4,5 % d'eau.

EXEMPLE DE REFERENCE 21

Une solution de 1,15 g d'acide fluoro-7 méthyl-1 (méthyl-3 pipérazinyl-1)-8 oxo-4 dihydro-1,4 benzo[b]naphtyridine-1,8 carboxylique-3 dans 1,35 cm3 d'acide formique à 98 et 3,25cm3 de formaldéhyde en solution aqueuse à 30 % est chauffée à une température voisine de 100°C pendant 2 heures. Le mélange réactionnel est concentré sous pression réduite (20 kPa) à 50°C puis additionné de 5 cm3 d'eau, la solution obtenue est amenée à pH 7 par addition de 0,5 cm3 de potasse aqueuse 2 N et chauffée à une température voisine de 100°C pendant 2 minutes. Le produit qui cristallise est essoré à 20°C, lavé par 2 fois 10 cm3 d'eau. Le produit brut obtenu est recristallisé 2 fois dans 10 cm3 de diméthylformamide à chaque fois. On obtient 0,55 g d'acide (diméthyl-3,4 pipérazinyl-1)-8 fluoro-7 méthyl-1 oxo-4 dihydro-1,4 benzo[b]naphtyridine-1,8 carboxylique-3 sous forme d'un solide jaune fondant à 306-308°C.

EXEMPLE DE REFERENCE 22

L'acide (diméthyl-3,4 pipérazinyl-1)-8 éthyl-1 fluoro-7 oxo-4 dihydro-1,4 benzo[b]naphtyridine-1,8 carboxylique-3 est préparé dans les conditions de l'exemple de référence 21, mais à partir de 2,3 g d'acide éthyl-1 fluoro-7 (méthyl-3 pipérazinyl-1)-8 oxo-4 dihydro-1,4 benzo[b]naphtyridine-1,8 carboxylique-3, de 2,26 cm3 d'acide formique à 98 % et de 5,6 cm3 de formaldéhyde en solution aqueuse à 30 %. On obtient 1,75 g d'acide (diméthyl-3,4 pipérazinyl-1)-8 éthyl-1 fluoro-7 oxo-4 dihydro-1,4 benzo[b]naphtyridine-1,8 carboxylique-3 sous forme d'un solide jaune fondant à 293-294°C.

EXEMPLE DE REFERENCE 23

L'acide cyclopropyl-1 (diméthyl-3,4 pipérazinyl-1)-8 fluoro-7 oxo-4 dihydro-1,4 benzo[b]naphtyridine- 1,8 carboxylique-3 est préparé dans les conditions de l'exemple de référence 21, mais à partir de 1,9 g d'acide cyclopropyl-1 fluoro-7 (méthyl-3 pipérazinyl-1)-8 oxo-4 dihydro-1,4 benzo[b]naphtyridine-1,8 carboxylique-3, de 1,38 cm3 d'acide formique et de 3,30 cm3 de formaldéhyde en solution aqueuse à 30%. Après une recristallisation du produit brut dans 50 cm3 d'éthanol, on obtient 1,3 g d'acide cyclopropyl-1 (diméthyl-3,4 pipérazinyl-1)-8 fluoro-7 oxo-4 dihydro-1,4 benzo[b]naphtyridine-1,8 carboxylique-3 sous forme d'un solide jaune fondant à 219°C.

EXEMPLE DE REFERENCE 24

Une suspension de 0,47 g d'acide cyclopropyl-1 difluoro-7,8 oxo-4 dihydro-1,4 benzo [b] naphtyridine-1,8 carboxylique-3 et de 0,6 g de méthyl-1 pipérazine dans 7 cm3 de diméthylsulfoxyde est chauffée à une température voisine de 80°C pendant 15 minutes. Le mélange réactionnel est versé dans 25 cm3 d'eau, additionné

de 9 cm3 d'acide chlorhydrique N. Le solide obtenu est essoré, lavé par 3 fois 5 cm3 d'eau. Après 1 recristallisation dans un mélange de 4,5 cm3 d'éthanol et de 4,5 cm3 de diméthylformamide on obtient 0,29 g d'acide cyclopropyl-1 fluoro-7 (méthyl-4 pipérazinyl-1)-8 oxo-4 dihydro-1,4 benzo [b] naphtyridine-1,8 carboxylique-3 sous forme d'un solide jaune fondant à 250°C.

EXEMPLE DE REFERENCE 25

Une suspension de 2 g d'acide difluoro-7,8 méthoxy-1 oxo-4 dihydro-1,4 benzo [b] naphtyridine-1,8 carboxylique-3 dans 2,8 g de pipérazine et 40 cm3 de diméthylsulfoxyde est agitée pendant 15 minutes à une température voisine de 40°C. Après refroidissement à environ 20°C, le mélange réactionnel est versé dans 150 cm3 d'eau, additionné de 27,75 cm3 d'acide méthanesulfonique 2N. Un très faible insoluble est éliminé par filtration sur silice diatomée. La solution obtenue est additionnée de 15 cm3 de potasse aqueuse 2N. Le précipité formé est essoré, lavé par 3 fois 15 cm3 d'eau, repris par 100 cm3 de diméthylformamide et chauffé sous agitation pendant 10 minutes à une température voisine de 150°C. La suspension est refroidie à environ 100°C ; l'insoluble est essoré, repris par 100 cm3 d'éthanol, chauffé à une température voisine de 75°C, pendant 1 heure. L'insoluble est essoré à environ 50°C et lavé par 40 cm3 du même solvant, à la même température que précédemment. On obtient 1,8 g d'acide fluoro-7 méthoxy-1 oxo-4 (pipérazinyl-1)-8 dihydro-1,4 benzo [b] naphtyridine-1,8 carboxylique-3 sous forme d'un solide brun fondant à 298-300°C, solvaté par 2,4 % d'eau.

EXEMPLE DE REFERENCE 26

Une suspension de 0,93 g d'acide difluoro-7,8 méthoxy-1 oxo-4 dihydro-1,4 benzo [b] naphtyridine-1,8 carboxylique-3 dans 0,6 g de méthyl-1 pipérazine et 20 cm3 de diméthylsulfoxyde est chauffée à une température voisine de 80°C pendant 5 minutes. Après refroidissement à environ 20°C, le mélange réactionnel est versé dans 30 cm3 d'eau, additionné de 1,5 cm3 d'acide méthanesulfonique 2N, essoré, lavé par 3 fois 5 cm3 d'eau. Après recristallisation dans 30 cm3 de diméthylformamide à 30 % d'éthanol, on obtient 0,55 g d'acide fluoro-7 méthoxy-1 (méthyl-4 pipérazinyl-1)-8 oxo-4 dihydro-1,4 benzo [b] naphtyridine-1,8 carboxylique-3 sous forme d'un solide brun fondant à 270°C.

EXEMPLE DE REFERENCE 27

Une suspension de 2 g d'acide chloro-8 fluoro-7 méthyl-1 oxo-4 dihydro-1,4 benzo [b] naphtyridine-1,8 carboxylique-3 dans 7,44 g de diméthyl-2,2 pipérazine et 20 cm3 de pyridine est chauffée à une température voisine de 115°C pendant 44 heures. En opérant comme décrit précédemment à l'exemple de référence 1, on obtient 1,6 g d'acide (diméthyl-3,3 pipérazinyl-1)-8 fluoro-7 oxo-4 dihydro-1,4 benzo [b] naphtyridine-1,8 carboxylique-3 sous forme d'un solide jaune solvaté par 4,9 % d'eau, fondant à 362-365°C.

EXEMPLE DE REFERENCE 28

Une suspension de 4 g d'acide trifluoro-7,8,9 méthyl-1 oxo-4 dihydro-1,4 benzo [b] naphtyridine-1,8 carboxylique-3 dans 60 cm3 de diméthylsulfoxyde et 3 g de méthyl-1 pipérazine est chauffée à 80°C pendant 1 heure et demie. Après refroidissement à environ 20°C, on ajoute 150 cm3 d'eau. La solution obtenue est additionnée de 18 cm3 d'acide acétique à 10 %. Le précipité formé est essoré, lavé par 3 fois 50 cm3 d'eau et recristallisé dans 50 cm3 de diméthylformamide. On obtient 4 g d'acide difluoro-7,9 méthyl-1 (méthyl-4 pipérazinyl-1)-8 oxo-4 dihydro-1,4 benzo [b] naphtyridine-1,8 carboxylique-3 sous forme d'un solide jaune fondant à 316°C.

EXEMPLE DE REFERENCE 29

Une suspension de 2 g d'acide trifluoro-6,7,8 méthoxy-1 oxo-4 dihydro-1,4 benzo [b] naphtyridine-1,8 carboxylique-3 et de 2,8 g de pipérazine dans 40 cm3 de diméthylsulfoxyde est chauffée, sous agitation à environ 50°C pendant 45 minutes. Après refroidissement à une température voisine de 20°C, la suspension obtenue est versée dans 100 cm3 d'eau, additionnée de 9,22 g d'acide méthanesulfonique. Un léger insoluble est éliminé par filtration sur silice diatomée. Le filtrat est additionné de 32 cm3 de potasse aqueuse 2N. Le précipité obtenu est essoré, lavé par 3 fois 50 cm3 d'eau et recristallisé dans 80 cm3 de diméthylformamide. On obtient 1,4 g d'acide difluoro-7,9 méthoxy-1 oxo-4 (pipérazinyl-1)-8 dihydro- 1,4 benzo [b] naphtyridine- 1,8 carboxylique-3 sous forme d'un solide jaune fondant à 305-308°C.

EXEMPLE DE REFERENCE 30

Une suspension de 1,2 g d'acide chloro-8 fluoro-7 (fluoro-2 éthyl)-1 oxo-4 dihydro-1,4 benzo [b] naphtyridine-1,8 carboxylique-3 dans 12 cm3 de pyridine et 3,52 g de méthyl-1 pipérazine est chauffée, sous agitation, à une température voisine de 110°C pendant 6 heures. Après traitement dans les conditions décrites à l'exemple de référence 3, on obtient 0,6 g d'acide fluoro-7 (fluoro-2 éthyl)-1 (méthyl-4 pipérazinyl-1)-8 oxo-4 dihydro-1,4 benzo [b] naphtyridine-1,8 carboxylique-3 sous forme d'un solide jaune fondant à 306-308°C.

La présente invention concerne également les compositions pharmaceutiques destinées à un usage local utilisables en médecine humaine ou vétérinaire qui contiennent comme produit actif au moins un produit de formule générale (I) dans laquelle R est autre qu'un radical protecteur, à l'état pur (sous forme libre ou sous forme de sel) ou sous forme d'une association avec un ou plusieurs diluants ou adjuvants compatibles et pharmaceutiquement acceptables.

Comme compositions solides à usage local peuvent être utilisées des poudres, des crèmes, des pommades, des gels. Dans ces compositions, le produit actif de formule générale (I) est mélangé à un ou plusieurs diluants ou adjuvants inertes tels que le lactose, les dérivés de la cellulose ou le talc par exemple. Ces compositions peuvent également comprendre d'autres substances comme par exemple des acides gras et leurs dérivés ou des corps gras d'origine animale, végétale ou synthétique.

Comme compositions liquides peuvent être utilisées les émulsions pharmaceutiquement acceptables pour l'usage local, des solutions, des suspensions contenant des diluants inertes tels que l'eau, des huiles (huile de paraffine, huile de vaseline, huile d'olive, ...) des esters organiques ... Ces compositions peuvent, en outre, contenir des adjuvants tels que des agents mouillants, émulsifiants, dispersants ou stabilisants.

Les compositions peuvent également être préparées sous forme de compositions solides qui peuvent être dissoutes au moment de l'emploi.

Les compositions contenant un produit de formule générale (I) sont particulièrement utiles dans le traitement des infections cutanées à staphylocoques.

D'une manière générale, les compositions contiennent des concentrations de 0,1 ‰ à 1 %.

L'exemple suivant illustre une composition à usage local contenant un produit de formule générale (I).

EXEMPLE

| | | |
|---|---|---|
| - acide cyclopropyl-1 difluoro-7,8 oxo-4 dihydro-1,4 benzo[b]naphtyridine-1,8 carboxylique-3 ............. | | 1 g |
| - oxyde de zinc ........................................ | | 1,5 g |
| - talc ........................................ | q.s.p.. | 100 g |

Enfin, les produits de formule générale (I) peuvent aussi être utilisés comme agents de conservation ou de désinfection des matières organiques ou minérales. Notamment dans l'industrie des colorants, des matières grasses, du papier, du bois, des polymères ou encore dans l'industrie textile, l'industrie alimentaire ou le traitement des eaux. Il est également entendu que les compositions destinées à la conservation ou à la désinfection et renfermant un produit de formule générale (I) à l'état pur ou sous forme d'association avec des diluants ou adjuvants compatibles, entrent aussi dans le cadre de la présente invention.

## Revendications

### Revendications pour les Etats contractants suivants : AT, BE, CH, DE, DK, FR, GB, IT, LI, LU, NL, SE

**1.** Un dérivé de la benzo[b]naphtyridine-1,8 caractérisé en ce qu'il répond à la formule générale :

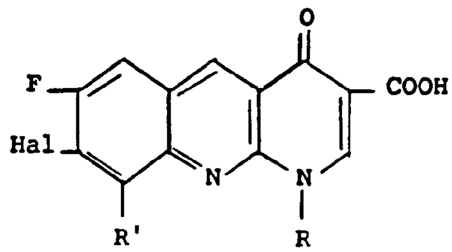

dans laquelle :

- R représente un atome d'hydrogène, un radical alcoyle, fluoroalcoyle, cycloalcoyle contenant 3 à 6 atomes de carbone, alcoyloxy, alcoylamino ou représente un radical protecteur d'amine, et soit Hal représente un atome de fluor de chlore ou de brome, et R' représente un atome d'hydrogène, soit les symboles Hal et R' représentent simultanément des atomes de fluor, étant entendu que les radicaux alcoyle cités ci-dessus sont droits ou ramifiés et contiennent 1 à 4 atomes de carbone, ses sels métalliques ainsi que ses sels d'addition avec les bases azotées.

2. Un dérivé de la benzo [b] naphtyridine-1,8 selon la revendication 1, caractérisé en ce que :
   - R représente un atome d'hydrogène, un radical alcoyle contenant 1 à 4 atomes de carbone, un radical fluoroéthyle, cyclopropyle, méthoxy ou méthylamino et
   soit Hal représente un atome de fluor ou de chlore et R' représente un atome d'hydrogène,
   soit Hal et R' représentent simultanément des atomes de fluor,
   ainsi que ses sels métalliques et ses sels d'addition avec les bases azotées.

3. Un dérivé selon la revendication 1, constitué de l'acide difluoro-7,8 méthyl-1 oxo-4 dihydro-1,4 benzo [b] naphtyridine-1,8 carboxylique-3, ainsi que ses sels métalliques et ses sels d'addition avec les bases azotées.

4. Un dérivé selon la revendication 1, constitué de l'acide chloro-8 fluoro-7 méthyl-1 oxo-4 dihydro-1,4 benzo[b]naphtyridine-1,8 carboxylique-3, ainsi que ses sels métalliques et ses sels d'addition avec les bases azotées.

5. Un dérivé selon la revendication 1, constitué de l'acide méthyl-1 oxo-4 trifluoro-7,8,9 dihydro-1,4 benzo[b]naphtyridine-1,8 carboxylique-3, ainsi que ses sels métalliques et ses sels d'addition avec les bases azotées.

6. Un dérivé selon la revendication 1, constitué de l'acide difluoro-7,8 méthoxy-1 oxo-4 dihydro-1,4 benzo [b]naphtyridine-1,8 carboxylique-3, ainsi que ses sels métalliques et ses sels d'addition avec les bases azotées.

7. Un dérivé selon la revendication 1, constitué de l'acide cyclopropyl-1 difluoro-7,8 oxo-4 dihydro-1,4 benzo[b]naphtyridine-1,8 carboxylique-3, ainsi que ses sels métalliques et ses sels d'addition avec les bases azotées.

8. Procédé de préparation d'un dérivé de la benzo[b]naphtyridine-1,8 selon la revendication 1, caractérisé en ce que l'on transforme l'ester de formule générale :

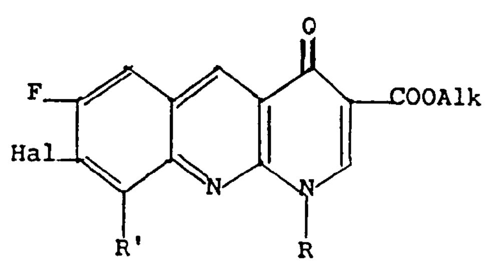

dans laquelle Hal et R' sont définis comme dans la revendication 1, R est un atome d'hydrogène, un radical

alcoyle, fluoroalcoyle, cycloalcoyle contenant 3 à 6 atomes de carbone, alcoyloxy ou alcoylamino ou alcoylamino protégé (les radicaux alcoyle contenant 1 à 4 atomes de carbone en chaine droite ou ramifiée) et Alk est un radical alcoyle contenant 1 à 4 atomes de carbone en chaîne droite ou ramifiée, par toute méthode connue pour obtenir un acide à partir d'un ester, sans toucher au reste de la molécule, puis le cas échéant élimine le groupement protecteur du radical alcoylamino, ou bien met en place un radical protecteur d'amine lorsque l'on veut obtenir un dérivé de la benzo[b]naphtyridine-1,8 pour lequel R a cette signification, et transforme éventuellement le produit obtenu en un sel métallique ou en un sel d'addition avec une base azotée.

**9.** Composition caractérisée en ce qu'elle contient au moins un dérivé selon la revendication 1, pour lequel R est défini comme dans la revendication 1 à l'exception de représenter un radical protecteur d'amine, à l'état pur ou en association avec un ou plusieurs diluants ou adjuvants compatibles.

**Revendications pour les Etats contractants suivants : ES, GR**

**1.** Procédé de préparation d'un dérivé de la benzo[b]naphtyridine-1,8 de formule générale :

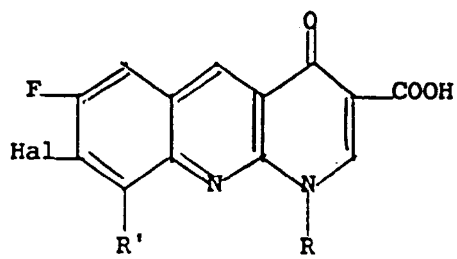

dans laquelle :

- R représente un atome d'hydrogène, un radical alcoyle, fluoroalcoyle, cycloalcoyle contenant 3 à 6 atomes de carbone, alcoyloxy, alcoylamino ou représente un radical protecteur d'amine, et soit Hal représente un atome de fluor de chlore ou de brome, et R' représente un atome d'hydrogène, soit les symboles Hal et R' représentent simultanément des atomes de fluor, étant entendu que les radicaux alcoyle cités ci-dessus sont droits ou ramifiés et contiennent 1 à 4 atomes de carbone, ainsi que ses sels métalliques et ses sels d'addition avec les bases azotées, caractérisé en ce que l'on transforme l'ester de formule générale :

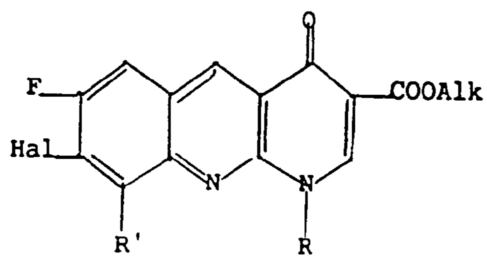

dans laquelle Hal et R' sont définis comme ci-dessus, R est un atome d'hydrogène, un radical alcoyle, fluoroalcoyle, cycloalcoyle contenant 3 à 6 atomes de carbone, alcoyloxy ou alcoylamino ou alcoylamino protégé (les radicaux alcoyle contenant 1 à 4 atomes de carbone en chaine droite ou ramifiée) et Alk est un radical alcoyle contenant 1 à 4 atomes de carbone en chaîne droite ou ramifiée, par toute méthode connue pour obtenir un acide à partir d'un ester, sans toucher au reste de la molécule, puis le cas échéant élimine le groupement protecteur du radical alcoylamino, ou bien met en place un radical protecteur d'amine lorsque l'on veut obtenir un dérivé de la benzo[b]naphtyridine-1,8 pour lequel R a cette signification, et transforme éventuellement le produit obtenu en un sel métallique ou en un sel d'addition avec une base azotée.

**Patentansprüche**

**Patentansprüche für folgende Vertragsstaaten : AT, BE, CH, DE, DK, FR, GB, IT, LI, LU, NL, SE**

1. Benzo[b]-1,8-naphthyridinderivat, dadurch gekennzeichnet, daß es der allgemeinen Formel

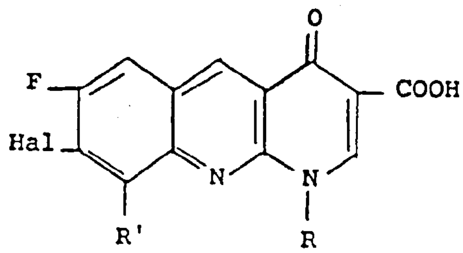

entspricht, worin

- R ein Wasserstoffatom, einen Alkyl-, Fluoralkyl-, Cycloalkyl- mit 3 bis 6 Kohlenstoffatomen, Alkoxy-, Alkylaminorest bedeutet oder eine Aminoschutzgruppe darstellt und entweder Hal für ein Fluor-, Chlor- oder Bromatom steht und R' ein Wasserstoffatom bedeutet, oder die Symbole Hal und R' gleichzeitig Fluoratome darstellen mit der Maßgabe, daß die vorstehend genannten Alkylreste geradkettig oder verzweigt sind und 1 bis 4 Kohlenstoffatome aufweisen, deren Metallsalze sowie Additionssalze mit Stickstoffbasen.

2. Benzo[b]-1,8-naphthyridinderivat gemäß Anspruch 1, dadurch gekennzeichnet, daß

- R für ein Wasserstoffatom, einen Alkylrest mit 1 bis 4 Kohlenstoffatomen, einen Fluorethyl-, Cyclopropyl-, Methoxy- oder Methylaminorest steht und
entweder Hal ein Fluor- oder Chloratom bedeutet und R' für ein Wasserstoffatom steht,
oder Hal und R' gleichzeitig-Fluoratome wiedergeben,
sowie deren Metallsalze und deren Additionssalze mit Stickstoffbasen.

3. Derivat gemäß Anspruch 1, bestehend aus der 7,8-Difluor-1-methyl-4-oxo-1,4-dihydro-benzo[b]-1,8-naphthyridin- 3-carbonsäure sowie aus deren Metallsalzen und deren Additionssalzen mit Stickstoffbasen.

4. Derivat gemäß Anspruch 1, bestehend aus der 8-Chlor-7-fluor-1-methyl-4-oxo-1,4-dihydro-benzo[b]-1,8-naphthyridin-3-carbonsäure sowie aus deren Metallsalzen und deren Additionssalzen mit Stickstoffbasen.

5. Derivat gemäß Anspruch 1, bestehend aus der 1-Methyl-4-oxo-7,8,9-trifluor-1,4-dihydro-benzo[b]-1,8-naphthyridin-3-carbonsäure sowie aus deren Metallsalzen und deren Additionssalzen mit Stickstoffbasen.

6. Derivat gemäß Anspruch 1, bestehend aus der 7,8-Difluor-1-methoxy-4-oxo-1,4-dihydro-benzo[b]-1,8-naphthyridin-3-carbonsäure sowie aus deren Metallsalzen und deren Additionssalzen mit Stickstoffbasen.

7. Derivat gemäß Anspruch 1, bestehend aus der 1-Cyclopropyl-7,8-difluor-4-oxo-1,4-dihyro-benzo[b]-1,8-naphthyridin-3-carbonsäure sowie aus deren Metallsalzen und deren Additionssalzen mit Stickstoffbasen.

8. Verfahren zur Herstellung eines Benzo[b]-1,8-naphthyridinderivats gemäß Anspruch 1, dadurch gekennzeichnet, daß man den Ester der allgemeinen Formel

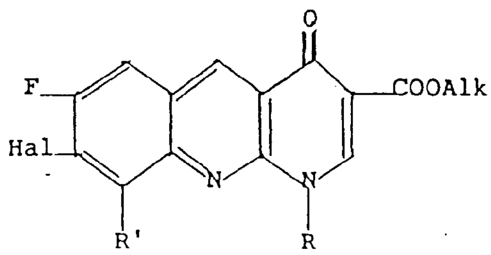

worin Hal und R' wie in Anspruch 1 definiert sind, R ein Wasserstoffatom, einen Alkyl-, Fluoralkyl-, Cycloalkyl- mit 3 bis 6 Kohlenstoffatomen, Alkoxy- oder Alkylamino- oder geschützten Alkylaminorest bedeutet (wobei die Alkylreste 1 bis 4 Kohlenstoffatome in gerader oder verzweigter Kette aufweisen) und Alk für einen Alkylrest mit 1 bis 4 Kohlenstoffatomen in gerader oder verzweigter Kette steht, nach jeder für die Erzielung einer Säure aus einem Ester ohne Beeinträchtigung des Restes des Moleküls bekannten Methode umwandelt, anschließend gegebenenfalls die Schutzgruppe des Alkylaminorestes entfernt oder eine Aminoschutzgruppe einführt, wenn man ein Benzo[b]-1,8-naphthyridinderivat erhalten möchte, worin R diese Bedeutung besitzt, und gegebenenfalls das erhaltene Produkt in ein Metallsalz oder ein Additionssalz mit einer Stickstoffbase überführt.

9. Zusammensetzung, dadurch gekennzeichnet, daß sie zumindest ein Derivat gemäß Anspruch 1, worin R mit Ausnahme der Bedeutung einer Aminoschutzgruppe wie in Anspruch 1 definiert ist, in reiner Form oder in Assoziation mit einem oder mehreren verträglichen Verdünnungsmitteln oder Adjuvantien enthält.

**Patentansprüche für folgende Vertragsstaaten : ES, GR**

1. Verfahren zur Herstellung eines Benzo[b]-1,8-naphthyridinderivats der allgemeinen Formel

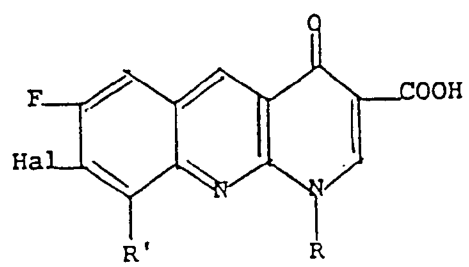

worin

- R ein Wasserstoffatom, einen Alkyl-, Fluoralkyl-, Cycloalkyl- mit 3 bis 6 Kohlenstoffatomen, Alkoxy-, Alkylaminorest bedeutet oder eine Aminoschutzgruppe darstellt und Hal entweder ein Fluor-, Chlor- oder Bromatom wiedergibt und R' ein Wasserstoffatom bedeutet, oder die Symbole Hal und R' gleichzeitig Fluoratome bedeuten mit der Maßgabe, daß die vorstehend angegebenen Alkylreste geradkettig oder verzweigt sind und 1 bis 4 Kohlenstoffatome aufweisen, sowie von dessen Metallsalzen und dessen Additionssalzen mit Stickstoffbasen, dadurch gekennzeichnet, daß man den Ester der allgemeinen Formel

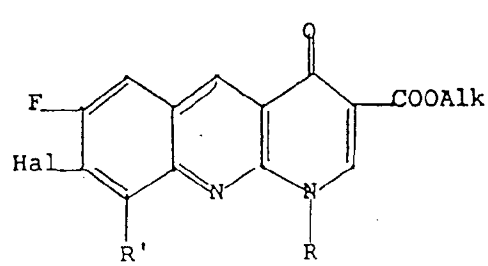

worin Hal und R' wie vorstehend definiert sind, R ein Wasserstoffatom, einen Alkyl-, Fluoralkyl-, Cycloalkyl- mit 3 bis 6 Kohlenstoffatomen, Alkoxy-, Alkylamino- oder geschützten Alkylaminorest bedeutet (wobei die Alkylreste 1 bis 4 Kohlenstoffatome in gerader oder verzweigter Kette aufweisen) und Alk für einen Alkylrest mit 1 bis 4 Kohlenstoffatomen in gerader oder verzweigter Kette steht, nach jeder für die Erzielung einer Säure aus einem Ester ohne Beeinträchtigung des Restes des Moleküls bekannten Methode umwandelt, wonach man gegebenenfalls die Schutzgruppe des Alkylaminorestes entfernt oder eine Aminoschutzgruppe einführt, wenn man ein Benzo[b]-1,8-naphthyridinderivat erhalten möchte, worin R diese Bedeutung besitzt und gegebenenfalls das erhaltene Produkt in ein Metallsalz oder ein Additionssalz mit einer Stickstoffbase überführt.

## Claims

### Claims for the following Contracting States : AT, BE, CH, DE, DK, FR, GB, IT, LI, LU, NL, SE

1. A 1,8-benzo[b]naphthyridine derivative, characterized in that it corresponds to the general formula:

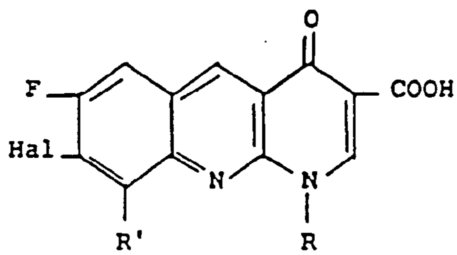

in which:

R represents a hydrogen atom, an alkyl, fluoroalkyl or cycloalkyl radical containing 3 to 6 carbon atoms, alkoxy or alkylamino or represents an amine-protective radical and

either Hal represents a fluorine, chlorine or bromine atom and R' represents a hydrogen atom, or the symbols Hal and R' simultaneously represent fluorine atoms,

it being understood that the alkyl radicals mentioned above are straight or branched and contain 1 to 4 carbon atoms,

its metal salts as well as its addition salts with nitrogen-containing bases.

2. A 1,8-benzo[b]naphthyridine derivative according to Claim 1, characterized in that:

R represents a hydrogen atom, an alkyl radical containing 1 to 4 carbon atoms, or a fluoroethyl, cyclopropyl, methoxy or methylamino radical and

either Hal represents a fluorine or chlorine atom and R' represents a hydrogen atom,

- or Hal and R' simultaneously represent fluorine atoms, as well as its metal salts and its addition salts with nitrogen-containing bases.

3. A derivative according to Claim 1, consisting of 7,8-difluoro-1-methyl-4-oxo-1,4-dihydro-1,8-benzo[b]naphthyridine-3-carboxylic acid, as well as its metal salts and its addition salts with nitrogen-containing bases.

4. A derivative according to Claim 1, consisting of 8-chloro-7-fluoro-1-methyl-4-oxo-1,4-dihydro-1,8-benzo[b]naphthyridine-3-carboxylic acid, as well as its metal salts and its addition salts with nitrogen-containing bases.

5. A derivative according to Claim 1, consisting of 1-methyl-4-oxo-7,8,9-trifluoro-1,4-dihydro-1,8-benzo[b]naphthyridine-3-carboxylic acid, as well as its metal salts and its addition salts with nitrogen-containing bases.

6. A derivative according to Claim 1, consisting of 7,8-difluoro-1-methoxy-4-oxo-1,4-dihydro-1,8-benzo[b]naphthyridine-3-carboxylic acid, as well as its metal salts and its addition salts with nitrogen-containing bases.

7. A derivative according to Claim 1, consisting of 1-cyclopropyl-7,8-difluoro-4-oxo-1,4-dihydro-1,8-benzo-

[b]naphthyridine-3-carboxylic acid, as well as its metal salts and its addition salts with nitrogen-containing bases.

8. Process for the preparation of a 1,8-benzo[b]naphthyridine derivative according to Claim 1, characterized in that the ester of general formula:

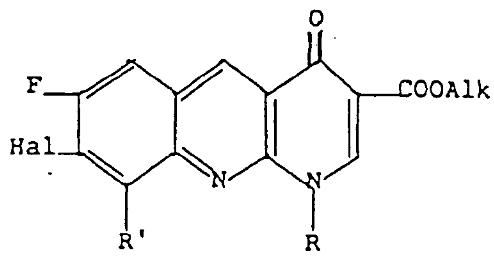

in which Hal and R' are defined as in Claim 1, R is a hydrogen atom, an alkyl, fluoroalkyl or cycloalkyl radical containing 3 to 6 carbon atoms, alkoxy or alkylamino or protected alkylamino (the alkyl radicals containing 1 to 4 carbon atoms as a straight or branched chain) and Alk is an alkyl radical containing 1 to 4 carbon atoms in a straight or branched chain, is converted by any known method for obtaining an acid from an ester, without affecting the remainder of the molecule, after which, where appropriate, the protective group of the alkylamino radical is removed or an amine-protective radical is introduced if it is desired to obtain a 1,8-benzo[b]naphthyridine derivative in which R has this meaning, and the product obtained is optionally converted to a metal salt or to an addition salt with a nitrogen-containing base.

9. Composition characterized in that it contains at least one derivative according to Claim 1, for which R is defined as in Claim 1 except for representing an amine-protective radical, in the pure state or in combination with one or more compatible adjuvants or diluents.

**Claims for the following Contracting States : ES, GR**

1. Process for the preparation of a 1,8-benzo[b]naphthyridine derivative of general formula:

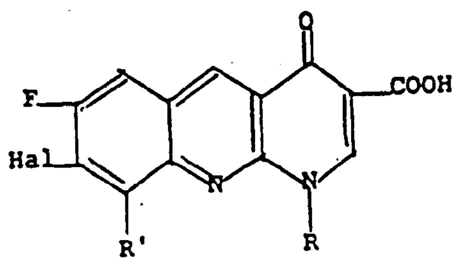

in which:

R represents a hydrogen atom, an alkyl, fluoroalkyl or cycloalkyl radical containing 3 to 6 carbon atoms, alkoxy or alkylamino or represents an amine-protective radical and

either Hal represents a fluorine, chlorine or bromine atom and R' represents a hydrogen atom, or the symbols Hal and R' simultaneously represent fluorine atoms,

it being understood that the alkyl radicals mentioned above are straight or branched and contain 1 to 4 carbon atoms, as well as its metal salts and its addition salts with nitrogen-containing bases, characterized in that the ester of general formula:

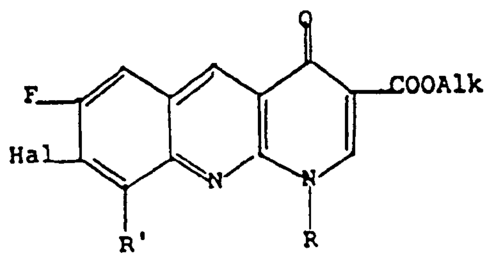

in which Hal and R' are defined as above, R is a hydrogen atom, an alkyl, fluoroalkyl or cycloalkyl radical containing 3 to 6 carbon atoms, alkoxy or alkylamino or protected alkylamino (the alkyl radicals containing 1 to 4 carbon atoms as a straight or branched chain) and Alk is an alkyl radical containing 1 to 4 carbon atoms in a straight or branched chain, is converted by any known method for obtaining an acid from an ester, without affecting the remainder of the molecule, after which, where appropriate, the protective group of the alkylamino radical is removed or an amine-protective radical is introduced if it is desired to obtain a 1,8-benzo[b]naphthyridine derivative in which R has this meaning, and the product obtained is optionally converted to a metal salt or to an addition salt with a nitrogen-containing base.